(19) Europäisches Patentamt
European Patent Office
Office européen des brevets

(11) Publication number: **0 619 299 A2**

# (12) EUROPEAN PATENT APPLICATION

(21) Application number: **94105517.0**

(22) Date of filing: **11.04.94**

(51) Int. Cl.5: **C07C 251/58**, C07D 295/12, A61K 31/15, A61K 31/445, A61K 31/495

(30) Priority: **09.04.93 HU 1004093**

(43) Date of publication of application: **12.10.94 Bulletin 94/41**

(84) Designated Contracting States: **AT BE CH DE DK ES FR GR IT LI NL SE**

(71) Applicant: **EGIS GYOGYSZERGYAR RT.**
**Kereszturi ut 30-38**
**H-1106 Budapest (HU)**

(72) Inventor: **Bajnogel, Judit, Dr.**
**Benkö I. u. 25**
**H-1151 Budapest (HU)**
Inventor: **Blasko, Gábor, Dr.**
**Molnár E. u. 21**
**H-1113 Budapest (HU)**
Inventor: **Budai, Zoltán, Dr.**
**Lukács u. 3**
**H-1023 Budapest (HU)**
Inventor: **Schmidt, Eva**
**Tárogato u. 64/b**
**H-1021 Budapest (HU)**
Inventor: **Egyed, András, Dr.**
**Ujvidék u. 58**
**H-1145 Budapest (HU)**
Inventor: **Fekete, Márton, Dr.**
**Fö u. 49**
**H-1027 Budapest (HU)**
Inventor: **Gacsályi, István**
**Baross u. 67**
**H-1021 Budapest (HU)**
Inventor: **Gyertyán, István, Dr.**
**Koronafürt u. 33**
**H-1165 Budapest (HU)**
Inventor: **Mezei, Tibor, Dr.**
**Borz u. 4**
**H-1221 Budapest (HU)**
Inventor: **Reiter, Klára, Dr. born Esses**
**Tövis u. 32/b**
**H-1022 Budapest (HU)**
Inventor: **Simig, Gyula, Dr.**
**Hollosy S. u. 25**
**H-1126 Budapest (HU)**
Inventor: **Szemerédi, Katalin, Dr.**
**Vörösvari u. 3**
**H-1035 Budapest (HU)**
Inventor: **Szirt, Enikö, born Kiszelly**
**Téglavetö u. 24**
**H-1105 Budapest (HU)**

(74) Representative: **Beszédes, Stephan G., Dr.**
**Patentanwalt**
**Postfach 11 68**
**D-85201 Dachau (DE)**

## (54) 1-(Phenyl)-3-[omega-(amino)-alkoxyimino]-alk-1-ene derivatives, a process for preparing them as well as medicaments containing them and the use of them.

(57) A subject-matter of the invention are 1-(Phenyl)-3-[ω-(amino)-alkoxyimino]-alk-1-ene derivatives of general formula

EP 0 619 299 A2

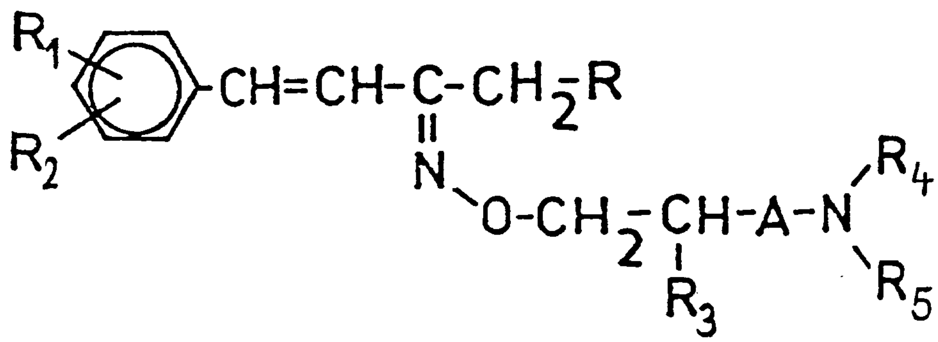

I,

wherein

R1 and R2 — independently each stands for hydrogen, halogen or a $C_{1-4}$ alkoxy group or together represent a 3,4-methylenedioxy group,

R — stands for $C_{1-8}$ alkyl,

$R_3$ — represents hydrogen, $C_{1-4}$ alkyl or hydroxy,

$R_4$ and $R_5$ — independently each represents hydrogen, a $C_{1-12}$ alkyl group, optionally substituted by hydroxy, or a $C_{2-12}$ alkenyl or $C_{3-6}$ cycloalkyl group,

or

$R_4$ and $R_5$ — together with the nitrogen atom to which they are attached form a 4- to 7-membered heterocyclic ring, optionally comprising an oxygen, sulfur or a further nitrogen atom, which latter optionally may carry a phenyl, benzyl or $C_{1-4}$ alkyl substituent

and

A — represents a valency bond or a $-CH_2$-group,

as well as stereo and optically active isomers and their mixtures, acid-addition salts and quaternary ammonium derivatives thereof.

Furthermore the invention is concerned with a process for preparing these compounds as well as medicaments containing them and the use of them for preparing medicaments, particularly having ulcus and gastric-acid-secretion inhibiting and anxiolytic effects.

The invention is concerned with novel basic oxime ethers with useful pharmacological, particularly ulcus and gastric--acid-secretion inhibiting and anxiolytic, effects, a process for preparing them as well as medicaments containing these compounds and the use of them.

Some alkoxyimino derivatives carrying a basic group as substituent have been known in the art.

The fluorene derivative "IPS-339" of formula

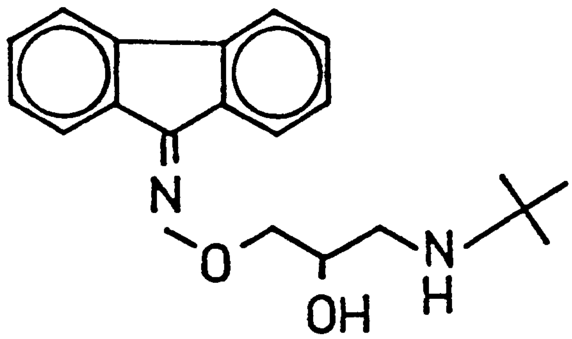

a

and the methyl cyclopropylketone derivative of formula

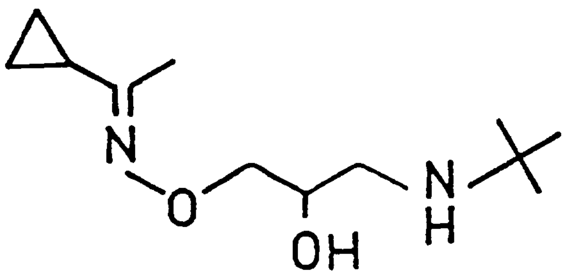

b,

known as Falintolol, exhibit beta-adrenergic blocking activity.

The compound of formula

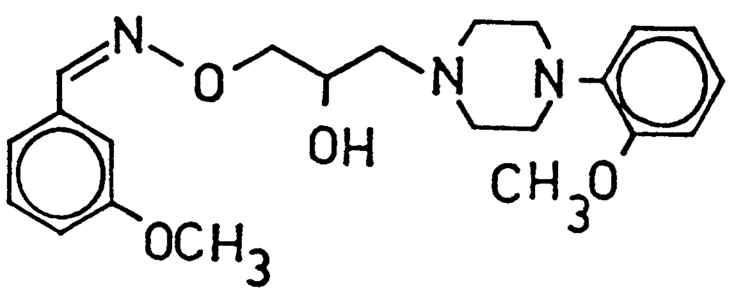

c,

known as Peradoxime, possesses blood pressure reducing activity.

Peraclopone of formula

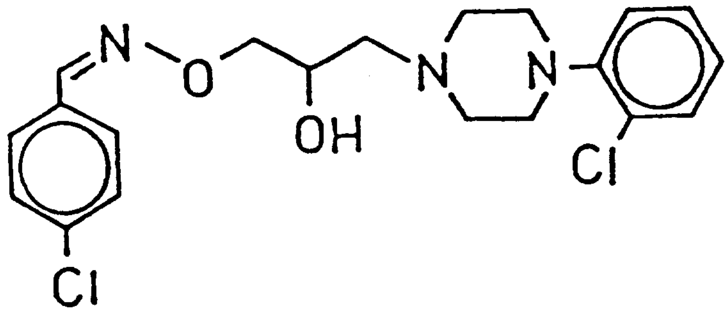

d,

reduces the level of lipids.

British patent specification No. 1,493,222 describes the compound of formula

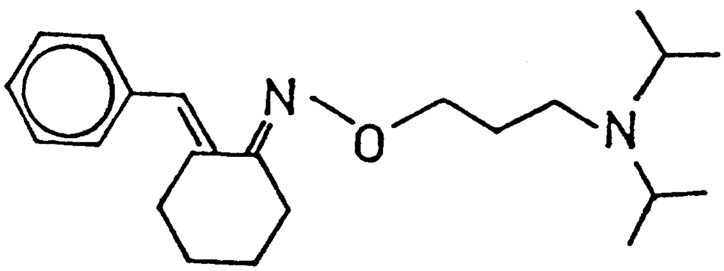

e

exhibiting local anaesthetic and antiparkinsonic activities.

Belgian patent specification 886,471 describes benzothiophene derivatives of formula

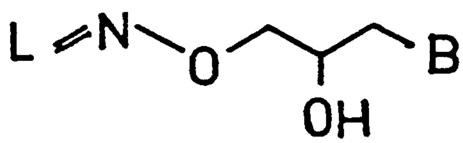

f,

wherein

- L represents a benzothiophene group and
- B stands for a secondary amino group.

The compounds exhibit beta-adrenergic blocking and antiarrhythmic activities.

Published PCT patent application 8,402,908 describes carbostyrylketoxime derivatives exhibiting not only beta-adrenergic blocking activity but antiglaucomic activity as well.

Belgian patent specification 838,440 describes beta-adrenergic blocking, blood-pressure reducing and cardiovascular compounds of formula f, wherein L represents a polycyclic ring, e. g. fluorene, indane, xanthane or tetrahydronaphthalene ring, or phenyl or naphthyl ketone and B always stands for a secondary amino group.

US patent specification 4,652,586 relates to compounds of formula f, wherein L is fluorene and B is a secondary amino group. The compounds reduce the inner pressure of eye and exhibit selective $\beta_2$-adrenergic antagonistic effect.

European patent specification No. 82,058 describes compounds of formula

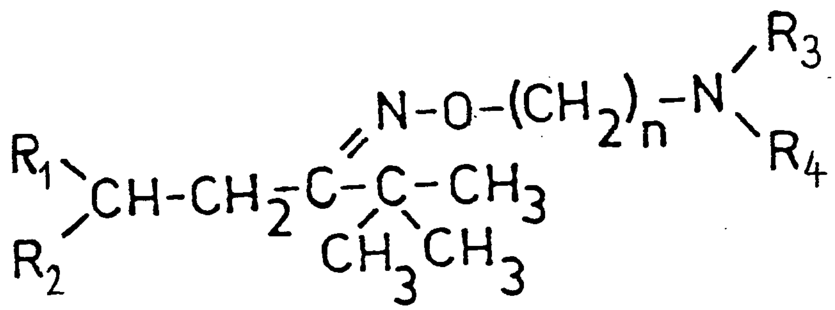

g ,

wherein $R_1$ represents cyclohexyl, phenyl, benzyl or $C_{1-4}$ alkyl, $R_2$ stands for piperidyl and $R_3$ and $R_4$ each represents straight or branched chained $C_{1-4}$ alkyl, or together with the nitrogen atom to which they are attached form a 4- to 7-membered saturated heterocyclic ring, optionally containing a further nitrogen or an oxygen atom, and n is an integer from 1 to 4. The compounds exhibit spasmolytic and antihistamine activities.

European patent specification 82,059 relates to oxime ether derivatives of formula

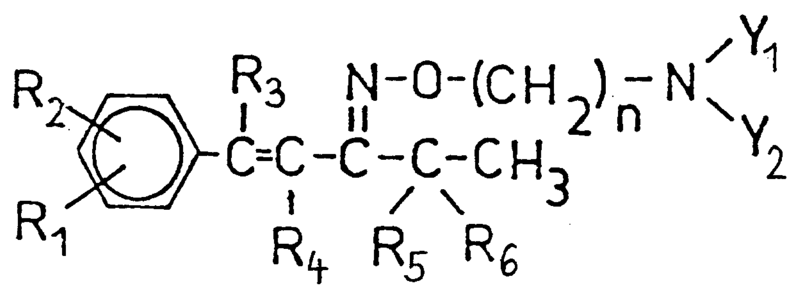

h ,

wherein $R_1$ and $R_2$ each represents hydrogen, halogen or $C_{1-4}$ alkyl or together form a methylenedioxy group, $R_3$ is hydrogen or phenyl, which latter carries a $C_{1-4}$ alkyl or halogen substituent, $R_4$ stands for hydrogen and $R_5$ is methyl or $R_4$ and $R_5$ together represent an alkyl chain containing 3 carbon atoms, $R_6$ denotes hydrogen or methyl, $Y_1$ and $Y_2$ each represents $C_{1-4}$ alkyl or together form an alkylene chain containing 4 to 7 carbon atoms, which may contain an oxygen or a nitrogen atom, and n is an integer from 1 to 4. The compound possess spasmolytic and antihistamine properties and are suitable for the treatment of circulatory disturbances.

The problem underlying to the invention is to create novel basic oxime ethers showing valuable pharmacological properties, particularly ulcus and gastric-acid-secretion inhibiting and anxiolytic effects, a process for preparing them as well as medicaments containing these compounds and the use of them.

Surprisingly this has been solved by the invention.

Thus a subject-matter of the invention are 1-(phenyl)-3-[ω-(amino)-alkoxyimino]-alk-1-ene derivatives of general formula

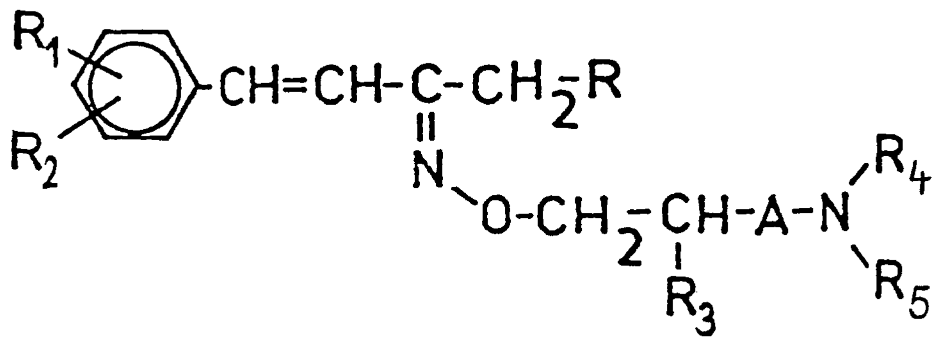

I,

wherein

| | |
|---|---|
| $R_1$ and $R_2$ | independently each stands for hydrogen, halogen or a $C_{1-4}$ alkoxy group or together represent a 3,4-methylenedioxy group, |
| R | stands for $C_{1-8}$ alkyl, |
| $R_3$ | represents hydrogen, $C_{1-4}$ alkyl or hydroxy, |
| $R_4$ and $R_5$ | independently each represents hydrogen, a $C_{1-12}$ alkyl group, optionally substituted by hydroxy, or a $C_{2-12}$ alkenyl or $C_{3-6}$ cycloalkyl group, |
| | or |
| $R_4$ and $R_5$ | together with the nitrogen atom to which they are attached form a 4- to 7-membered heterocyclic ring, optionally comprising an oxygen, sulfur or a further nitrogen atom, which latter optionally may carry a phenyl, benzyl or $C_{1-4}$ alkyl substituent |
| | and |
| A | represents a valency bond or a $-CH_2$-group, |

as well as stereo and optically active isomers and their mixtures, acid-addition salts and quaternary ammonium derivatives thereof.

The chemical structure of the 1-(phenyl)-3-[ω-(amino)-alkoxyimino]-alk-1-ene derivatives according to the invention is different from that of the Prior Art compounds and the activity thereof is different from that of the latter ones and surprising.

The term "alkyl group" used throughout the specification relates to straight or branched chained saturated aliphatic hydrocarbon groups having the given number of carbon atom(s), e. g. methyl, ethyl, propyl, isopropyl, (methyl)-ethyl, n-butyl or tert.-butyl groups. The term "alkenyl group" designates straight or branched chained alkenyl groups containing the given number of carbon atoms, e. g. vinyl, allyl, 2-(methyl) -allyl, prop-1-enyl, but-1-enyl, but-2-enyl or hex-2-enyl groups. The term "cycloalkyl group" relates to cycloalkyl groups having rings of 3 to 6 carbon atoms, e. g. cyclopropyl, cyclohexyl and cyclopentyl. The term "alkoxy group" relates to alkyl ether groups comprising 1 to 4 carbon atom(s), e. g. methoxy, ethoxy or tert.-butoxy groups. The term "halogen atom" encompasses all the four halogen atoms, that is fluorine, chlorine, bromine and iodine. The term "4- to 7-membered ring" relates to aromatic or partially or completely saturated heterocyclic groups which contain as [a] heteroatom(s) a nitrogen and optionally an oxygen, sulfur or further nitrogen atom, e. g. piperidyl, morpholinyl, piperazinyl, furyl, imidazolyl, pyridinyl, pyrazolyl, imidazolyl, hexamethyleneimino and hexahydroazepinyl groups, and the latter further heteroatom optionally may carry a phenyl, benzyl or $C_{1-4}$ alkyl substituent.

It is preferred that the $C_{1-4}$ alkyl group which may be represented by $R_3$ and/or the $C_{1-4}$ alkyl substituent by which the optional further nitrogen atom of the 4- to 7-membered heterocyclic ring which may be formed by $R_4$ and $R_5$ together with the nitrogen atom to which they are attached may be substituted and/or the $C_{1-4}$ alkoxy group(s) which may be represented by $R_1$ and/or $R_2$ is/are such having from 1 to 3, particularly 1 or 2, most particularly 1, carbon atom(s) and/or the $C_{1-12}$ alkyl group(s) which may be represented by $R_4$ and/or $R_5$ is/are such having from 1 to 8, particularly 1 to 4, more particularly 1 to 3, most particularly 1 or 2, above all 1, carbon atom(s) and/or the $C_{1-8}$ alkyl group which may be represented by R is such having from 1 to 7, particularly 1 to 6, most particularly 2 to 5, above all 2 to 4, carbon atom(s) and/or the $C_{2-12}$ alkenyl group(s) which may be represented by $R_4$ and/or $R_5$ is/are such having from 2 to 6, particularly 2 to 4, most particularly 2 or 3, above all 2, carbon atom(s) and/or the 4- to 7-membered heterocyclic ring which may be formed by $R_4$ and $R_5$ together with the nitrogen atom to which they are attached is such having from 5 to 7, particularly 5 or 6, carbon atoms, most particularly a morpholino, pyrrolidinyl, piperidinyl, piperazinyl, hexamethyleneimino or hexahydroazepinyl ring and/or the halogen atom(s) which may be represented by $R_1$ and/or $R_2$ is/are chlorine, fluorine and/or bromine, particularly the first one, and/or the $C_{3-6}$ cycloalkyl group(s) which may be represented by $R_4$ and/or $R_5$ is/are such having 3, 5 or 6 carbon atoms.

In branched chained alkyl groups which may be represented by R preferably the branching is on the carbon atom before the terminal one.

A particularly preferred group of compounds according to the invention are those in which

| | |
|---|---|
| $R_1$ and $R_2$ | independently each represents hydrogen or halogen, |
| $R_3$ | stands for hydrogen or hydroxy, |
| $R_4$ and $R_5$ | independently each represents $C_{1-4}$ alkyl |
| | or |
| $R_4$ and $R_5$ | together with the nitrogen atom to which they are attached form a piperidinyl or pyrrolidinyl group |
| | and |
| A and R | are as above defined. |

Particularly preferred compounds according to the invention are 1-[phenyl]-5-[methyl]-3-(E)-[2'-(dimethylamino)-ethoxyimino]-1-(E)-hexene, 1-[4'-(chloro)-phenyl]-6-[methyl]-3-(E)-[3''-(N-{piperidinyl})-propoxyimino]-1-(E)-hexene, 1-(4'-(chloro)-phenyl]-3-(E)-[2''-(N{piperidinyl})-ethoxyimino]-1-(E)-nonene, (R,S)-1-[4'-(chloro)-phenyl]-6-[methyl]-3-(E)-[2''-(hydroxy)-3''-(piperidin-1'''-yl)-propoxyimino]-1-(E)-heptene, 1-[4'-(fluoro)-phenyl]-6-[methyl]-3-(E)-[3''-(N-{piperidinyl})-propoxyimino]-1-(E)-heptene, 1-[4'-(chloro)-phenyl]-6-[methyl]-3-(Z)-[2''-(N-{methyl}-pyrrolidin-2'''-yl)-ethoxyimino]-1-(E)-heptene and 1-[4'-(chloro)-phenyl]-6-[methyl]-3-(E)-[3''-(N-{pyrrolidinyl})-propoxyimino]-1-(E)-heptene as well as stereo and optically active isomers, acid-addition salts and quaternary ammonium derivatives thereof.

Suitably the acid-addition salts of the 1-(phenyl)-3-[ω-(amino)-alkoxyimino]-alk-1-ene derivatives of formula I according to the invention are pharmaceutically acceptable ones. Advantageous acid-addition salts are those with hydrogen halides, sulfuric acid, phosphoric acid, tartaric acid, succinic acid, acetic acid, fumaric acid, maleic acid, methanesulfonic acid or propionic acid.

The 1-(phenyl)-3-[ω-(amino)-alkoxyimino]-alk-1-ene derivatives of formula I according to the invention may comprise one or two asymmetric carbon atoms depending on the character of the substituents; thus they can be in optically active forms, too. The invention covers all of the racemic or optically active forms of the compounds of general formula I according to the invention.

According to a further aspect of the invention there is provided for a process for preparing the compounds according to the invention which is characterized in that in a manner known per se

a) 1-(phenyl)-alk-1-ene-3-one or -3-thione derivatives of general formula

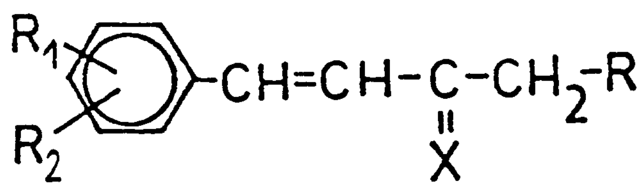

II,

wherein $R_1$, $R_2$ and R are as above defined and X stands for oxygen or sulfur, are reacted with substituted alkyl-0-hydroxylamines of general formula

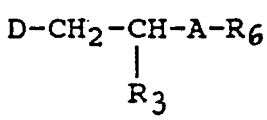

III,

wherein D represents a group of formula $H_2N$-0-,
$R_6$ stands for a group of formula

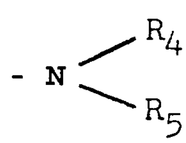

,

in which latter $R_4$ and $R_5$ are as above defined and $R_3$ and A are as above defined, or with the acid-addition salts thereof in the presence of a base

or

b) for preparing compounds of general formula I, wherein $R_3$ represents hydrogen or $C_{1-4}$ alkyl and R, $R_1$, $R_2$, $R_4$, and $R_5$ are as above defined, 1-(phenyl)-alk-1-ene-3-oxime derivatives of general formula

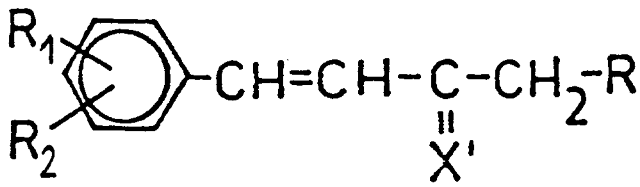 II',

wherein $R_1$ , $R_2$ and R are as above defined and X' stands for a group of formula = N - OH, or acid-addition salts thereof are reacted with substituted alkylhalides of general formula

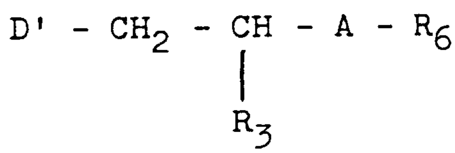 III'

wherein D' stands for halogen, $R_3$ represents hydrogen or $C_{1-4}$ alkyl, $R_6$ is a group of formula

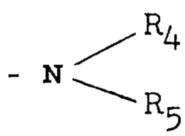 ,

in which latter $R_4$ and $R_5$ are as above defined and A is as above defined, or with acid-addition salts thereof in the presence of a basic condensing agent

or

c) for preparing compounds of general formula I, wherein $R_3$ represents hydroxy, A stands for a group of formula - $CH_2$ - and R, $R_1$ , $R_2$ , $R_4$ and $R_5$ are as above defined, 1-(phenyl)-alk-1-ene-3-oxime derivatives of general formula II', wherein X' represents a group of formula = N - 0H and $R_1$ , $R_2$ and R are as above defined, or acid-addition salts thereof are reacted with 2,3-(epoxy)-propoxyhalides of general formula

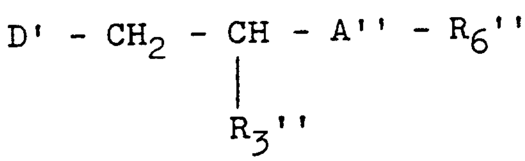 III'',

wherein D' represents halogen and A'', $R_6$'' and $R_3$'' together form a group of formula - $CH_2$ - 0 -, in the presence of a basic condensing agent, and the thus obtained 1-(phenyl)-3-[2',3'-(epoxy)-propoxyimino]-alk-1-enes of general formula

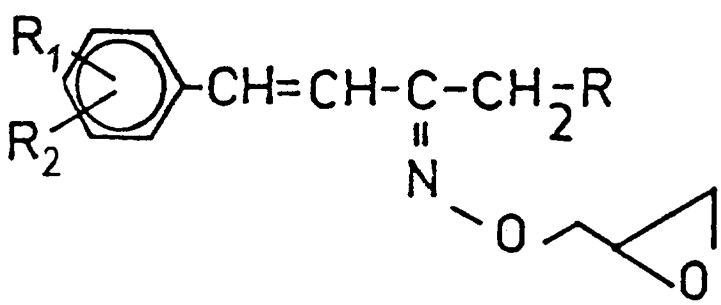 V,

wherein $R_1$ , $R_2$ and R are as above defined, are reacted with amines of general formula

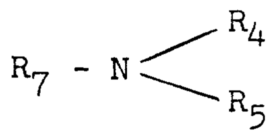

IV,

wherein $R_7$ stands for hydrogen and $R_4$ and $R_5$ are as above defined, or in the presence of a basic agent with acid-addition salts of the former

or

d) for preparing compounds of general formula I, wherein $R_3$ represents hydrogen or $C_{1-4}$ alkyl and R, $R_1$, $R_2$, $R_4$, $R_5$ and A are as above defined, 1-(phenyl)-alk-1-ene-3-oxime derivatives of general formula II', wherein X' represents a group of formula = N - 0H and $R_1$, $R_2$ and R are as above defined, or acid-addition salts thereof are reacted with α,ω-dihalogenalkanes of general formula

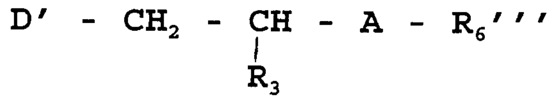

III''',

wherein D' stands for halogen, $R_3$ represents hydrogen or $C_{1-4}$ alkyl, $R_6$''' means halogen and A is as above defined, in the presence of a basic condensing agent and the thus obtained 1-(phenyl)-3-[ω-(halogen)-alkoxyimino]-alk-1-ene derivatives of general formula

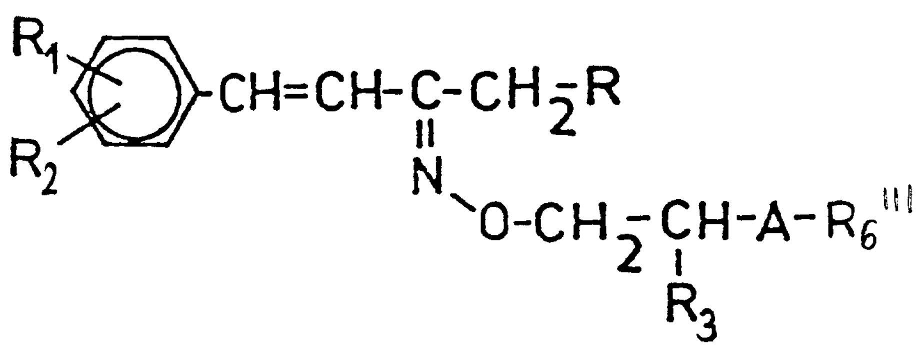

V',

wherein $R_1$, $R_2$, $R_3$, R and A are as above defined and $R_6$''' is as defined for formula III''', are reacted with amines of general formula

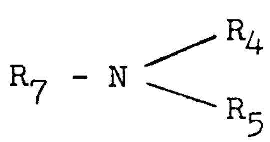

IV,

wherein $R_7$ stands for hydrogen and $R_4$ and $R_5$ are as above defined, or with acid-addition salts thereof in the presence of a basic condensing agent

and optionally in a manner known per se 1-(phenyl)-3-[ω-(amino)-alkoxyimino]-alk-1-ene derivatives of general formula I thus obtained are converted into acid-addition salts or quaternary ammonium derivatives thereof or salts of 1-(phenyl)-3-[ω-(amino)-alkoxyimino]-alk-1-ene derivatives of general formula I thus obtained are converted into the free 1-(phenyl)-3-[ω-(amino)-alkoxyimino]-alk-1-ene derivative bases and/or into other acid-addition salts and/or mixtures or racemates of the above compounds of formula I are separated to the stereo and/or optically active isomers of the above compounds of formula I or the optically active isomers of the above compounds of formula I are racemised.

The reaction of the 1-(phenyl)-alk-1-ene-3-one or -3-thione derivatives of general formula II with the substituted alkyl-0-hydroxylamines of general formula III of variant a) of the process according to the invention is preferably carried out in inert solvents or in mixtures of such solvents, particularly in an aliphatic alcohol, e. g. methanol or ethanol, most preferably in the latter, pyridine or triethylamine or in a mixture

thereof. As a basic condensing agent preferably an organic base, particularly pyridine, piperidine or morpholine or a mixture thereof is used. If the solvent is an organic base, it may serve as condensing agent, too. The reaction temperature may be varied within wide ranges. The reaction can be performed even at room temperature, but according to the experiments the optimal reaction rate can be achieved at the boiling point of the reaction mixture.

In the reaction of the 1-(phenyl)-alk-1-ene-3-oxime derivatives of general formula II' with the substituted alkylhalides of general formula III' of variant b) of the process according to the invention as basic condensing agents preferably alkali amides, particularly sodium amide, alkali hydrides, particularly sodium hydride, alkali metals, particularly sodium, alkali alcoholates or alkali hydroxides, particularly sodium hydroxide, or organic bases, particularly pyridine, or mixtures thereof, such as mixtures of sodium hydroxide and potassium hydroxyde in the ratio by weight of 9 : 1 to 1 : 9, are used. Preferably the reaction is carried out in inert solvents or mixtures of such solvents, particularly in an aliphatic alcohol, most particularly ethanol, aromatic hydrocarbon, most particularly benzene or a homologue thereof, such as toluene or xylene, aliphatic or cyclic ether, most particularly dibutyl ether or tetrahydrofuran, or dimethylformamide or dimethyl acetamide or in a mixture thereof. Further examples of suitable solvents are dimethyl acetamide and diethylacetamide. The temperature of the reaction can vary within wide ranges, generally from 25 °C to the boiling point of the applied solvent. It is preferable to carry out the reaction at temperatures of from 70 to 130 °C.

Suitably the reaction of the 1-(phenyl)-alk-1-ene-3-oxime derivatives of general formula II' with the 2,3-(epoxy)-propoxyhalides of general formula III'' or $\alpha,\omega$-dihalogenalkanes of general formula III''', respectively, of variant c) or d), respectively, of the process according to the invention is carried out in inert or relatively inert solvents. Preferably as inert solvents alcohols, particularly ethanol, or benzene, toluene, xylene or aliphatic cyclic ethers or dimethylformamide or mixtures thereof are used. As basic condensing agent preferably an alkali amide, particularly sodium amide, an alkali hydride, particularly sodium hydride, an alkali hydroxide, particularly sodium hydroxide, an alkali alcoholate or an organic base, particularly pyridine, is used. Also alkali metals can be used as condensing agents. In this latter case alcohols, such as ethanol or propanol, are the most preferred solvents. If an alkali hydroxide is used as a basic condensing agent, water may also serve as solvent [in such a case water is a relatively inert solvent in case of variant c), as it reacts with the epoxy ring after a longer reaction time and at higher temperatures]. The temperature of the reaction can vary within wide ranges, generally from room temperature to the boiling point of the mixture, the latter being preferred.

Also the reaction of the 1-(phenyl)-3-[2',3'-(epoxy)-propoxyimino]-alk-1-enes of general formula V with the amines of general formula IV of variant c) of the process according to the invention is preferably carried out in an inert solvent, e. g. in an alcohol, advantageously aliphatic alcohol, particularly ethanol, or acetonitrile, or an aliphatic or cyclic ether, e. g. dioxane or tetrahydrofuran or a mixture thereof, but when using amines of general formula IV having high boiling point also it can be completed even without using any solvent. In such cases an excess of the applied amine serves as solvent. As basic agents applied in case of using acid-addition salts of the amines of formula IV preferably tri-$C_{1-4}$-alkylamines, such as triethylamine, are used. The reaction temperature may be varied within a wide interval. It can be room temperature to the boiling point of the reaction mixture an optimal rate of reaction being achieved at the boiling point of the reaction mixture. Analogous considerations apply for the second step of variant d) of the process according to the invention.

For converting the 1-(phenyl)-3-[$\omega$-(amino)-alkoxyimino]-alk-1-ene derivatives of general formula I into acid-addition salts advantegeously hydrogen halides, sulfuric acid, phosphoric acid, tartaric acid, succinic acid, acetic acid, fumaric acid, maleic acid, methanesulfonic acid or propionic acid can be used. For preparing quaternary ammonium compounds the 1-(phenyl)-3-[$\omega$-(amino)-alkoxyimino]-alk-1-ene derivatives of general formula I can be reacted with reactants suitable for quaternarization, e. g. with alkyl halides.

If the compounds or intermediates having one or two asymmetric carbon atoms are prepared in the form of a diastereomeric mixture, they can be separated into racemic or optically active isomers in a manner known per se, e. g. by fractional distillation, crystallization, chromatography or by forming diastereomeric salts with the aid of optically active acids such as tartaric acid, dibenzoyltartaric acid or camphorsulfonic acid.

The 1-(phenyl)-alk-1-ene-3-one or -3-thione derivatives of general formula II usable as starting substances are known and can be prepared e. g. according to the method described in Org. Syn. Coll. 2 [1943], 167. The substituted alkyl-0-hydroxylamines of general formula III can be prepared as described in J. Pharm. Sci. 58 [1969], 138.

The 1-(phenyl)-alk-1-ene-3-oxime derivatives of general formula II' usable as starting compounds for variants b), c) and d) of the process according to the invention can be prepared e. g. according to the

method described in Org. Synth. Coll. Vol. II [1943], 70.

The substituted alkylhalides of general formula III', the 2,3-(epoxy)-propoxyhalides of general formula III'' and the $\alpha,\omega$-dihalogenalkanes of general formula III''' usable as starting substances for variants b), c) and d) of the process according to the invention can be prepared as described in Helv. Chim. Acta 41 [1958], 1 072 to 1 108 or in Beilstein 17, 1/v 20.

As already mentioned, the 1-(phenyl)-3-[$\omega$-(amino)-alkoxyimino]-alk-1-ene derivatives according to the invention possess a considerable gastric-acid-secretion and ulcus inhibiting (antiulceric) and anxiolytic activity. They are also potent inhibitors of the gastric $H^+/K^+$-ATP-ase. At the same time their toxicity is only slight, so that they can be used as active ingredients in pharmaceutical compositions.

Hence by the invention also there are provided for medicaments which are characterized in that they contain as [an] active ingredient(s) at least one compound according to the invention, suitably in admixture with at least one inert solid and/or liquid pharmaceutical carrier and/or diluent and/or excipient.

The medicaments according to the invention can be in the form of pharmaceutical preparations. These can be prepared by methods known per se and thus suitably by admixing the 1 or more 1-(phenyl)-3-[$\omega$-(amino)-alkoxyimino]-alk-1-ene derivatives according to the invention with 1 or more inert solid and/or liquid carrier(s) and/or diluent(s) and/or excipient(s) and bringing the mixture to a galenic form.

Preferably the pharmaceutical preparations of the invention are in the form of tablets or dragées for oral administration. The daily dose of the 1-(phenyl)-3-[$\omega$-(amino)-alkoxyimino]-alk-1-ene derivatives according to the invention can vary within wide ranges depending on several factors, e. g. on the activity of the active ingredient(s), the patient's condition and age and the severity of the disease. The oral dose is generally 1 to 300 mg/day. It has to be stressed that these doses are only of informative character and the administered dose must always be determined by the physician therapeutist.

The 1-(phenyl)-3-[$\omega$-(amino)-alkoxyimino]-alk-1-ene derivatives according to the invention also can be in the form of solution or suspension preparations.

The 1-(phenyl)-3-[$\omega$-(amino)-alkoxyimino]-alk-1-ene derivatives according to the invention can serve as active ingredients for preparing medicaments having ulcus and gastric-acid-secretion inhibiting and anxiolytic effects, for example, useful in the treatment of disorders caused by hyperacidity (gastric or duodenal ulcer), in the treatment of gastric mucosa caused by antiphlogistics (glucocorticoids, salicylic acid derivatives) or for the mitigation of gastric disorders caused by alcoholism.

Hence a further subject-matter of the invention is the use of the compounds according to the invention for preparing medicaments, particularly having ulcus and gastric-acid-secretion inhibiting and anxiolytic effects.

The compounds according to the invention can be used administering to the patient an effective amount of them.

The biological activity of the 1-(phenyl)-3-[$\omega$-(amino)-alkoxyimino]-alk-1-ene derivatives according to the invention is shown by the following tests.

I. Toxicity

The test was carried out according to the method of Lichtfield and Wilcoxon (Lichtfield, J.T. and Wilcoxon, F.W.: J. Pharmacol. Exp. Ther., 96, 99 [1949]) by using white mice belonging to the CFLP strain and weighing 10 to 22 g, 10 animals per dose. The test compounds were administered orally in a volume of 20 $cm^3$/kg. After treatment the animals were observed for a period of 14 days. The results are summarized in Table I.

Table I

Toxicity on mice

| Compound | | $LD_{50}$ mg/kg | |
|---|---|---|---|
| Designation | Example | i.p. | p.o. |
| 1-[Phenyl]-5-[methyl]-3-(E)-[2'-(dimethylamino)-ethoxyimino]-1-(E)-hexene | 17 | 100 to 300 | >1 000 |
| 1-[4'-(Chloro)-phenyl]-6-[methyl]-3-(E)-[3''-(N-{piperidinyl})-propoxyimino]-1-(E)-hexene | 9 | 160 | >1 000 |
| 1-[4'-(Chloro)-phenyl]-3-(E)-[2''-(N-{piperidinyl})-ethoxyimino]-1-(E)-nonene | 3 | 100 to 300 | 500 to 1 000 |
| 1-[4'-(Chloro)-phenyl]-6-[methyl]-3-(E)-[3''-(hexahydro-1''H-azepinyl)-propoxyimino]-1-(E)-heptene | 34 | 30 to 100 | > 1 000 |
| (R,S)-1-[4'-(Chloro)-phenyl]-6-[methyl]-3-(E)-[3''-(cyclohexylamino)-2''-(hydroxy)-propoxyimino]-1-(E)-heptene | 35 | 30 to 100 | > 1 000 |
| (R,S)-1-[4'-(Chloro)-phenyl]-6-[methyl]-3-(E)-[2''-(hydroxy)-3''-(piperidin-1'''-yl)-propoxyimino]-1-(E)-heptene | 36 | 100 to 300 | >1 000 |
| 1-[4'-(Fluoro)-phenyl]-6-[methyl]-3-(E)-[3''-(N-{piperidinyl})-propoxyimino]-1-(E)-heptene | 37 | 100 to 300 | 500 to 1 000 |
| 1-[4'-(Chloro)-phenyl]-6-[methyl]-3-(Z)-[2''-({N-methyl}-pyrrolidin-2'''-yl)-ethoxyimino]-1-(E)-heptene | 39 | 100 to 300 | 1 000 |

Table I continued

| Compound | | $LD_{50}$ mg/kg | |
|---|---|---|---|
| Designation | Example | i.p. | p.o. |
| 1-[4'-(Chloro)-phenyl]-6-[methyl]-3-(E)-[3''-(N-{pyrrolidinyl})-propoxyimino]-1-(E)-heptene | 40 | 30 to 100 | > 1 000 |
| 1-[4'-(Bromo)-phenyl]-6-[methyl]-3-(E)-{3''-[N-(piperidinyl)]-propoxyimino}-1-(E)-heptene | 41 | 100 to 300 | > 1 000 |

II. Gastric ulcer inhibiting (antiulceric) activity

Test methods:

1. $H^+/K^+$-ATP-ase inhibiting on pig's stomach.
The test was carried out according to the method of Rabon and Sachs [E.C. Rabon, W.B.I. and G. Sachs: Preparation of gastric $H^+/K^+$-ATP-ase. Methods in Enzymology, **157**, 649-651 (1988)]. The activity of the prepared enzyme was measured both in the presence and in the absence of $K^+$ ions. The difference in the liberation of phosphorus representing the activity of the enzyme was measured.
2. The gastric-acid-secretion test was carried out on rats according to the method of Shay et al. [Shay, H., Komarov, S.A., Fels, S.S., Meranze, D., Gruenstein, M., Siplet, H.: Gastroenterology 5, 43-61 (1945)]. The liberated gastric acid content was determined by titration 4 hours after the ligature of the duodenum.
3. The cytoprotective effect was determined on rats according to the method of Robert [Robert, A.: Cytoprotection by prostaglandins. Gastroenterology **77**, 761-767 (1979)]. Rats weighing 200 to 250 g were used as test animals. 1 $cm^3$ of abs. ethanol was introduced to the stomach to produce an erosion of the stomach wall. The length and frequency of the lesions (erosion index and frequency) were measured and the percentage inhibition of lesion formation compared with vehicle-treated animals was calculated.
4. Measurement of $^{14}C$-aminopyrine accumulation by parietal cells. Gastric mucosal cells were prepared from rat stomach. Wistar rats (130-160 g) were killed by decapitation, the stomachs were rapidly excised and their contents were washed out with saline. The stomachs were then everted and filled with 2.5 mg/ml of pronase®-containing buffer. These sacs were incubated for 60 minutes at 37 °C in carbogen-gassed medium. This incubation was followed by gentle stirring at room temperature for 45 minutes by a magnetic stirrer in order to disperse the cells from the mucosa of the everted stomachs digested only from the serosal side. The viability of the cells was determined by trypan-blue exclusion test. The percentage of the parietal cells was determined on the basis of their morphological characteristics.

Acid production of the cells prepared in this way could be induced by cyclic AMP, histamine (in the presence of 3-isobutyl-1-methylxanthine) or carbachol. The acid production was assessed by measuring the accumulation of $^{14}C$-aminopyrine. The undissociated weak base can penetrate into the acid-containing compartments of the cells. In the acidic compartment the aminopyrine dissociates and for the dissociated form the membrane is impermeable. Thus, the distribution of $^{14}C$-aminopyrine between the extracellular and intracellular spaces is an indirect quantitative index for the cellular acid production [W. Schepp, J. Schmidtler, C. Tatge, V. Schusdziarra and M. Classen: Am. J. Physiol. 259 (Gastrointest. Liver Physiol. 22) G646-G654 (1990)].

Results

1. The compounds according to the invention are potent inhibitors of the so-called proton pump ($H^+/K^+$-ATP-ase) in concentrations of about 30 $\mu$M or lower. This indicates that they are useful in the treatment of gastric ulcer.

Table II

Inhibition of $H^+/K^+$-ATP-ase on partially purified pig's microsome specimen

| Compound | | $IC_{50}$ (µM) |
|---|---|---|
| Designation | Example | |
| 1-[Phenyl]-5-[methyl]-3-(E)-[2'-(dimethylamino)-ethoxyimino]-1-(E)-hexene | 17 | >30 |
| 1-[4'-(Chloro)-phenyl]-6-[methyl]-3-(E)-[3''-(N-{piperidinyl})-propoxyimino]-1-(E)-hexene | 9 | 12 |
| 1-[4'-(Chloro)-phenyl]-3-(E)-[2''-(N-{piperidinyl})-ethoxyimino]-1-(E)-nonene | 3 | 10 |
| 1-[4'-(Chloro)-phenyl]-6-[methyl]-3-(E)-[3''-(hexahydro-1''H-azepinyl)-propoxyimino]-1-(E)-heptene | 34 | 13 |
| (R,S)-1-[4'-(Chloro)-phenyl]-6-[methyl]-3-(E)-[2''-(hydroxy)-3''-(piperidin-1'''-yl)-propoxyimino]-1-(E)-heptene | 36 | 21 |
| 1-[4'-(Fluoro)-phenyl]-6-[methyl]-3-(E)-[3''-(N-{piperidinyl})-propoxyimino]-1-(E)-heptene | 37 | 17 |
| 1-[4'-(Chloro)-phenyl]-6-[methyl]-3-(Z)-[2''-({N-methyl}-pyrrolidin-2'''-yl)-ethoxyimino]-1-(E)-heptene | 39 | 10 |
| 1-[4'-(Bromo)-phenyl]-6-[methyl]-3-(E)-{3''-[N-(piperidinyl)]-propoxyimino}-1-(E)-heptene | 41 | 7 |
| 1-[4'-(Chloro)-phenyl]-6-[methyl]-3-(E)-[3''-(N-{pyrrolidinyl})-propoxyimino]-1-(E)-heptene | 40 | 9 |

According to the examinations the compounds according to the invention inhibit the aminopyrine accumulation by rat's parietal cell occuring during the stimulation of gastric-acid-secretion. The $ED_{50}$ of the compound according to Example 9 is 0.05 µM.

2. The cytoprotective effect of the compounds is significant, and according to the literature (D.E. Wilson: Therapeutic aspects of prostaglandins in the treatment of peptic ulcer disease. Dig. Dis. Sci. 31 [1986], 42-46S) this is a favourable characteristic considering the potential therapeutic utility.

Table III

Gastric-acid-secretion inhibiting and cytoprotective effects

| Compound Designation | Example | Acid-secretion inhibition, $ED_{50}$ mg/kg, p.o. | Ethanol erosion $ED_{50}$ mg/kg, p.o. | Ratio of gastric-acid-secretion inhibition to erosion inhibition |
|---|---|---|---|---|
| 1-[Phenyl]-5-[methyl]-3-(E)-[2'-(dimethylamino)-ethoxyimino]-1-(E)-hexene | 17 | 166 | 2.7 | 61.4 |
| 1-[4'-(Chloro)-phenyl]-6-[methyl]-3-(E)-[3''-(N-{piperidinyl})-propoxyimino]-1-(E)-hexene | 9 | 107 | 5.9 | 18.1 |
| 1-[4'-(Chloro)-phenyl]-3-(E)-[2''-(N-{piperidinyl})-ethoxyimino]-1-(E)-nonene | 3 | 50 to 150 | 2.5 | > 20 |
| (R,S)-1-[4'-(Chloro)-phenyl]-6-[methyl]-3-(E)-[2"-(hydroxy)-3"-(piperidin-1'''-yl)-propoxyimino]-1-(E)-heptene | 36 | 30 to 100 | 3 to 10 | >10 |
| 1-[4'-(Fluoro)-phenyl]-6-[methyl]-3-(E)-[3"-(N-{piperidinyl})-propoxyimino]-1-(E)-heptene | 37 | 37.7 | 1.3 | 29.0 |
| 1-[4'-(Chloro)-phenyl]-6-[methyl]-3-(Z)-[2"-({N-methyl}-pyrrolidin-2'''-yl)-ethoxyimino]-1-(E)-heptene | 39 | < 100 | 0,9 | < 100 |
| 1-[4'-(Chloro)-phenyl]-6-[methyl]-3-(E)-[3''-(N-{pyrrolidinyl})-propoxyimino]-1-(E)-heptene | 40 | < 100 | 2.0 | < 50 |

Table III continued

| Compound | | Acid-secretion inhibition, $ED_{50}$ mg/kg, p.o. | Ethanol erosion $ED_{50}$ mg/kg, p.o. | Ratio of gastric-acid-secretion inhibition to erosion inhibition |
|---|---|---|---|---|
| Designation | Example | | | |
| 5-Methoxy-2-(4-methoxy-3,5-dimethyl-2-pyridylmethyl-sulfinyl)-benzimidazole [Omeprazole] | Reference substance A | 3.9 | 4.5 | 0.9 |
| 1-Cyan-2-methyl-3-[2-(5-methyl-4-imidazolyl)-methyl-thioethyl]-guanidine [Cimetidine] | Reference substance B | 59.1 | 100 to 200 | 0.3 to 0.6 |
| 11-[(4-Methyl-1-piperazinyl)-acetyl]-5H-pyrido[2,3-b][1,4]benzodiazepin-6(11H)-one [Pirenzipine] | Reference substance C | 7.9 | 18.6 | 0.4 |
| Basic aluminium salt of saccharose-hydrogen-sulfate [Sucralfate] | Reference substance D | - | 69.0 | - |

From the above test results it can be established that the compounds according to the invention are only slightly toxic and at the same time they inhibit the gastric-acid secretion at doses 3 to 30 times lower than the toxic doses ($LD_{50}$). From the low $ED_{50}$ values obtained in the ethanol erosion test it can be seen that the cytoprotective effect of the test compounds according to the invention is highly superior to their gastric-acid-secretion activity. The compounds according to the invention are somewhat less potent

inhibitors of the gastric-acid-secretion than omeprazole or pirenzepine, but considering the inhibition of the erosion of the stomach wall produced by ethanol they are superior to both reference substances. From this fact it appears that the mechanism of the effect of the compounds according to the invention is different from that of the known substances exerting an antiulcer activity. The difference demonstrated by the gastric-acid-secretion inhibition/erosion inhibition ratios is very favourable, especially in the treatment of human diseases wherein the injury of the stomach wall occurs simultaneously with a decreased acid production (e.g. gastric disorders caused by alcoholism).

**III. Anxiolytic, sedative activity**

The compounds according to the invention exert an anxiolytic effect and at the same time they are devoid of any sedative, spontaneous motor activity decreasing side-effect. Certain compounds, when applied in high doses, exhibit a slight antipsychotic effect, too.

**1. Anxiolytic effect**

**1.1. Vogel test (drinking conflict test)**

The anxiolytic effect was tested by using the method of Vogel et al. Male Wistar rats of 160 to 180 g body weight were kept free of food and drinking water for 24 and 48 hours, respectively. Test and carrier substances were administered intraperitoneally half an hour before testing. Animals within the experimental chamber were provided with drinking water through an inserted tube. After the animals' each twenty lapping for water the device emitted a 2 mA intensity electric shock through the drinking tube. During 5 minutes the shocks tolerated by the animals in order to quench their thirst were counted. The effect of treatment was expressed as the % increase of the tolerated shocks. The minimum effective dose (MED) was determined for each test compound [Volge, J.R., Beer, B., Clody, D.E.: Psychopharmacologia (Berl.) **21**, 1 (1971)]. The data thus obtained are summarized in Table IV.

Table IV

| Compound | | MED (mg/kg) |
|---|---|---|
| Designation | Example | |
| 1-[Phenyl]-3-(E)-[3'-(dimethylamino)-propoxyimino-1-(-E)-hexene | 21 | 0.03 |
| (R,S)-1-[4'-(Chloro)-phenyl]-3-(E)-[3''-(dimethylamino)--2''-(methyl)-propoxyimino]-1 | 19 | 10.0 |
| -(E)-pentene (R,S)-1-[Phenyl]-3-(E)-[3'-(dimethylamino)-2'-(methyl)--propoxyimino]-1-(E)-pentene | 18 | 1.0 |
| 1-[Phenyl]-5-[methyl]-3-(E)-[2'-(dimethylamino)-ethoxyimino]-1-(E)-hexene | 17 | 30.0 |
| 1-[4'-(Chloro)-phenyl]-6-[methyl]-3-(E)-[2''-(dimethylamino)-ethoxyimino]-1-(E)-heptene | 15 | 3.0 |
| 1-[Phenyl]-3-(E)-[2'-(dimethylamino)-ethoxyimino]-1-(-E)-pentene | 14 | 10:0 |
| 1-[4'-(Chloro)-phenyl]-6-[methyl]-3-(E)-[3''-(dimethylamino)-propoxyimino]-1-(E)-heptene | 13 | > 30.0 |
| 1-Phenyl-5-methyl-3-(E)-[2-(N-piperidinyl)-ethoxyimino]-1-(E)-hexene | 10 | 0.1 |
| 1-[Phenyl]-6-[methyl]-3-(E)-[3'-(dimethylamino)-propoxyimino]-1-(E)-heptene | 5 | > 30.0 |
| 1-[4'-(Chloro)-phenyl]-3-(E)-[2''-(dimethylamino)-ethoxyimino]-1-(E)-pentene | 7 | 3.0 |
| 1-[Phenyl]-3-(E)-[2'-(diethylamino)-ethoxyimino]-1-(E)--hexene | 1 | 30.0 |
| 1-[4'-(Chloro)-phenyl]-6-[methyl]-3-(E)-[3''-(N-piperidinyl)-propoxyimino-1-(E)-hexene | 9 | > 30.0 |
| 2-(Methyl)-2-(propyl)-trimethylendicarbamate [Meprobamate] | Reference substance E | 25 |

During the above-specified test the compounds according to the invention proved to be more active than the reference compound or showed an activity in the same order of magnitude as the latter one.

The compounds of Examples 21 and 10, the most active members in the structural group, were examined in detail in other anxiolytic models, too.

**1.2. Elevated plus maze test on rat**

The test was carried out with the aid of a wooden plus-shaped maze elevated to a height of 50 cm. Two arms - opposite to one another - were enclosed up to a height of 40 cm along their longer sides and at the end. The other two arms were without walls (open arms). The central 15x15 cm part was open. Male rats belonging to the Sprague Dawley strain and weighing 220 to 260 g were used as test animals. After pretreatment lasting 60 minutes the animals were placed into the central part of the maze. During the 5 minutes observation period the following parameters were recorded:

- time spent on the open arms
- time spent on the closed arms
- number of open arm entries
- number of closed arm entries

The drug effect was expressed as percent increase of the time spent on the open arms and number of

open arm entries. The minimum effective dose (MED) which caused a significant increase of the time spent on the open arms was calculated for every substance [Pelow, S., Chopin, P., File, S.E., Briley, M.: J. Neurosci. Methods **14**, 149-167 (1985)].

The data are shown in Table V.

Table V

| Compound | | MED (mg/kg) p.o. |
|---|---|---|
| Designation | Example | |
| 1-[Phenyl]-3-(E)-[3'-(dimethylamino)-propoxyimino]-1-(E)-hexene | 21 | 1.0 |
| 1-Phenyl-5-methyl-3-(E)-[2-(N-piperidinyl)-ethoxyimino]-1-(E)-hexene | 10 | 10.0 |
| 2-(Methyl)-2-(propyl)-trimethylendicarbamate [Meprobamate] | Reference substance E | > 10.0 |

In order to establish whether the anxiolytic activity is accompanied by a sedative side-effect, the influence of the compounds on the spontaneous motor activity was also investigated.

**2. Sedative effect**

**2.1. Effect on the spontaneous motor activity**

The test was carried out according to the method of Borsy et al. Groups consisting of 3 mice each were treated orally with different doses of the compounds to be tested. Then the test animals were placed into a 10-channel Dews system equipment. In this equipment the number of interruptions of infrared beam within 30 minutes was counted. From these data 50 % inhibiting doses ($IC_{50}$) were determined with the aid of a line of regression [Borsy, J., Csányi, E., Lázár, I.: Arch. Int. Pharmacodyn. **124**, 1 (1960)]. The data are shown in Table VI.

Table VI

| Compound | | $ID_{50}$ |
|---|---|---|
| Designation | Example | (mg/kg) |
| 1-[Phenyl]-3-(E)-[3'-(dimethylamino)-propoxyimino]-1-(E)-hexene | 21 | >100 |
| (R,S)-1-[4'-(Chloro)-phenyl]-3-(E)-[3''-(dimethylamino)-2''-(methyl)-propoxyimino]-1--(E)-pentene | 19 | >100 |
| (R,S)-1-[Phenyl]-3-(E)-[3'-(dimethylamino)--2'-(methyl)-propoxyimino]-1-(E)--pentene | 18 | 100 mg/kg, inhibition of 54% |
| 1-[Phenyl]-5-[methyl]-3-(E)-[2'-(dimethylamino)-ethoxyimino]-1-(E)-hexene | 17 | > 100 |
| 1-[4'-(Chloro)-phenyl]-6-[methyl]-3-(E)-[2"--(dimethylamino)-ethoxyimino]-1-(E)-heptene | 15 | > 100 |
| 1-[Phenyl]-3-(E)-[2'-(dimethylamino)-ethoxy-imino]-1-(E)-pentene | 14 | > 100 |
| 1-[4'-(Chloro)-phenyl]-6-[methyl]-3-(E)--[3"-(dimethylamino)-propoxyimino]-1-(E)--heptene | 13 | > 100 |
| 1-[Phenyl]-5-[methyl]-3-(E)-[2-(N-piperidinyl)-ethoxyimino]-1-(E)-hexene | 10 | > 100 |
| 1-[Phenyl]-6-[methyl]-3-(E)-[3'-(dimethylamino)-propoxyimino]-1-(E)-heptene | 5 | > 100 |
| 1-[4'-(Chloro)-phenyl]-3-(E)-[2"-(dimethylamino)-ethoxyimino]-1-(E)-pentene | 7 | > 100 |
| 1-[Phenyl]-3-(E)-[2'-(diethylamino)-ethoxy-imino]-1-(E)-hexene | 1 | > 100 |
| 1-[4'-(Chloro)-phenyl]-6-[methyl]-3-(E)-[3"--(N-{piperidinyl})-propoxyimino]-1-(E)-hexene | 9 | > 100 |

Table VI continued

| Compound: Designation | Example | $ID_{50}$ (mg/kg) |
|---|---|---|
| 7-(Chlor)-N-(methyl)-5-(phenyl)-3H-1,4-benzodiazepin-2-ylamine-4-oxide [Chlorodiazepoxide] | Reference substance F | 56 |
| 7-(Chlor)-1-(methyl)-5-(phenyl)-1H-1,4-benzodiazepin-2(3H)-one [Diazepam] | Reference substance G | 23 |

From the data of the above Table it can be seen that the compounds according to the invention do not possess a sedative, spontaneous motor activity affecting effect in the anxiolytic dose interval.

## 3. Antipsychotic effect

Conditioned avoidance response inhibition

The antipsychotic (neuroleptic) effect was measured by the inhibition of the learned conditioned avoidance reflex. The male Wistar rats, used for the study, were of 120-150 g body weight at the commencement of learning. A shuttle box was used as experimental device; it consists of two parts, 24 x 24.5 x 23 cm each, separated by a wall with a 6 x 9 cm gate. In response to a suitable warning stimulus, in order to avoid the punishing (unconditioned) stimulus, animals within the box passed through the gate from one part to the other. The warning, i.e. conditioned stimulus (CS), a white light (1 Hz) blinking for 15 seconds, was applied at the place of the actual animal existence. The unconditioned stimulus (US), in form of 0.6 mA intensity electric shock, was randomly applied to the paw during the last 5 seconds of the conditioned stimulus. The animal's movement during the CS and US from one part of the box to the other, was defined as avoidance and escape responses, respectively. Both responses ceased the actual stimulus and stopped the trial. The time elapsed until the next trial (intertrial interval, ITI) was 15 seconds. While one experiment was carried out daily, an experiment consisted of 80 trials. Learning efficiency was expressed as percentage of the successful to the total avoidances. Effect of the neuroleptic drugs was examined in animals with stabilized conditioned reflexes and with at least 75 % learning efficiency. Dosing was carried out once a week, one hour before the measurement in the shuttle box. To calculate the neuroleptic effect (50 % inhibiting dose, $ID_{50}$), results obtained after the treatment were compared to those obtained on the previous day (controls). The obtained data are shown in Table VII.

Table VII

| Antipsychotic effect | | |
|---|---|---|
| Compound | | Inhibition of the conditioned reflex (%), 30 mg/kg p.o. |
| Designation | Example | |
| 1-[Phenyl]-3-(E)-[3'-(dimethylamino)-propoxyimino]-1-(-E)-hexene | 21 | $7^{++}$ |
| (R,S)-1-[4'-(Chloro)-phenyl]-3-(E)-[3''-(dimethylamino)--2''-(methyl)-propoxyimino]-1-(E)-pentene | 19 | 19 |
| (R,S)-1-[Phenyl]-3-(E)-[3'-(dimethylamino)-2'-(methyl)--propoxyimino]-1-(E)-pentene | 18 | 12 |
| 1-[Phenyl]-5-[methyl]-3-(E)-[2'-(dimethylamino)-ethoxy-imino]-1-(E)-hexene | 17 | $40^{++}$ |
| 1-[4'-(Chloro)-phenyl]-6-[methyl]-3-(E)-[2''-(dimethylamino)-ethoxyimino]-1-(E)-heptene | 15 | 11 |
| 1-[Phenyl]-3-(E)-[2'-(dimethylamino)-ethoxyimino]-1-(E-)-pentene | 14 | $18^{++}$ |
| 1-[4'-(Chloro)-phenyl]-6-[methyl]-3-(E)-[3''-(dimethylamino)-propoxyimino]-1-(E)-heptene | 13 | $12^{++}$ |
| 1-[Phenyl]-5-[methyl]-3-(E)-[2-(N-piperidinyl)-ethoxyimino]-1-(E)-hexene | 10 | $13^{++}$ |
| 1-[Phenyl)-6-[methyl]-3-(E)-[3'-(dimethylamino)-propoxyimino]-1-(E)-heptene | 5 | $16^{++}$ |
| 1-[4'-(Chloro)-phenyl]-3-(E)-[2''-(dimethylamino)-ethoxyimino]-1-(E)-pentene | 7 | $22^{++}$ |
| 1-[Phenyl)-3-(E)-[2'-(diethylamino)-ethoxyimino]-1-(E)--hexene | 1 | $10^{++}$ |
| 1-[4'-(Chloro)-phenyl]-6-[methyl]-3-(E)-[3''-(N-{piperidinyl})-propoxyimino]-1-(E)-hexene | 9 | $16^{++}$ |
| 10-[2'-(1''-⟨Methyl⟩-piperid-2''-yl)-ethyl]-2-[methylthio]--phenothiazine {Thioridazine} | Reference substance H | $ID_{50}$ = 108.0 |

$^{++}$ = $p < 0.05$ (test carried out with two samples)

Only one of the compounds according to the invention tested (the compound according to Example 17) exhibited a slight conditioned-reflex-inhibiting activity.

Summarized it can be established that the compounds according to the invention already in low concentrations are effective inhibitors of the enzyme responsible for the acid production. They possess a considerable gastric-acid-secretion inhibiting activity in vivo, too. The cytoprotective activity of compounds according to the invention is outstanding and independent of the proton pump inhibiting effect. The toxicological characteristics of the compounds according to the invention are favourable, as the $ED_{50}$ values are 100 to 1 000 times lower than the acute $LD_{50}$ values. Accordingly, new compounds exerting proton pump inhibiting and cytoprotective activities have been found, which

a) are chemically substantially different from the hitherto known molecules having similar effects, so that these properties could not be aforeseen on the basis of the chemical structure;

b) possess an enzyme-inhibiting activity being of a $\mu$M order of magnitude under the experimental conditions used and

c) have an outstanding cytoprotective effect which is independent of the proton pump inhibiting activity.

The compounds according to the invention showed psychotropic activity in some animal models. The most characteristic feature thereof is the anxiolytic activity without any influence on the spontaneous motor activity. This character is of basic importance considering the human therapy, as it indicates that the compounds according to the invention may be suitable for the treatment of different fear reactions, generalized anxiety disorders or post-traumatic stresses, without decreasing the vigilance and having a sedative potential.

The invention is further illustrated by the following Examples. In the thin layer chromatography all ratios indicated are by volume.

**Example 1**

**1-Phenyl-3-(E)-[2-(diethylamino)-ethoxyimino]-1-(E)-hexene**

1-Phenyl-1-(E)-hexen-3-one-(E)-oxime (18.5 g; 0.1 mole) is transformed into a salt with sodium hydride (4.8 g; 0.1 mole, 50 % oily dispersion) in the mixture of 30 $cm^3$ of dimethylformamide and 20 $cm^3$ of benzene, and this salt is condensed with 2-chloro-N,N-diethylethylamine (14.9 g; 0.11 mole) at a temperature of 40 to 60 °C. The stirring is continued until the oxime cannot be detected in the reaction mixture by thin layer chromatography (Kieselgel $GF_{254}$, eluent: 4:1 mixture of n-hexane and dioxane, UV). The reaction mixture is washed with water, extracted with 0.1N hydrogen chloride solution, precipitated with an aqueous ammonium hydroxide solution, extracted and evaporated.
Yield: 27.2 g (94.5 %) of yellow viscous oil.
2-(E)-Butenedioate (1/1) M.p.: 88 to 90 °C

| Analysis for the formula $C_{22}H_{32}N_2O_5$ (404.5): | | | |
|---|---|---|---|
| Calculated: | C % = 65.32 | H % = 7.97 | N % = 6.93 |
| Found: | C % = 65.53 | H % = 7.89 | N % = 6.91. |

UV: $\lambda_{max}$ = 291 nm ($\epsilon$ = 31203)

**Example 2**

**1-Phenyl-6-methyl-3-(E)-[2-(diethylamino)-ethoxy-imino]-1-(E)-heptene**

One proceeds as described in Example 1 except that instead of 1-phenyl-1-(E)-hexen-3-one-(E)-oxime 21.7 g (0.1 mole) of 1-phenyl-6-methyl-1-(E)-hepten-3-one-(E)-oxime are used.
Yield: 27.6 g (87.3 %) of yellowish brown oil.
2-(E)-Butenedioate (1/1) M.p.: 105 to 107 °C

| Analysis for the formula $C_{24}H_{36}N_2O_5$ (432.5): | | | |
|---|---|---|---|
| Calculated: | C % = 66.64 | H % = 8.39 | N % = 6.48 |
| Found: | C % = 66.53 | H % = 8.32 | N % = 6.44. |

UV: $\lambda_{max}$ = 288 nm ($\epsilon$ = 33744)

**Example 3**

**1-(4-Chlorophenyl)-3-(E)-[2-(N-piperidinyl)-ethoxyimino]-1-(E)-nonene**

1-(4-chlorophenyl)-1-(E)-nonen-3-one-(E)-oxime (26.5 g; 0.1 mole) is reacted with 1-(2-chloroethyl)-piperidine (16.2 g; 0.11 mole) in a solution of 100 g of 10 % sodium ethylate in ethanol at the boiling point of the reaction mixture. The reaction is continued until the starting oxime cannot be detected any more in the reaction mixture in the way as specified in Example 1. The mixture is then evaporated, the product is precipitated and filtered off.
Yield: 28.7 g (76.5 %) of yellow oil.
2-(E)-Butenedioate (1/1) M.p.: 114 °C

| Analysis for the formula $C_{26}H_{27}ClN_2O_5$ (493.0): | | | | |
|---|---|---|---|---|
| Calculated:<br>Found: | C % = 63.34<br>C % = 63.48 | H % = 7.56<br>H % = 7.52 | Cl% = 7.19<br>Cl% = 7.22 | N % = 5.68<br>N % = 5.73. |

UV: $\lambda_{max}$ = 289 nm ($\epsilon$ = 30576)

**Example 4**

**1-(4-Chlorophenyl)-6-methyl-3-(E)-[2-(N-piperidinyl)-ethoxyimino]-1-(E)-heptene**

One proceeds as described in Example 3 except that 1-(4-chlorophenyl)-6-methyl-1-(E)-hepten-3-one-(E)-oxime (26.5 g, 0.1 mole) is used as oxime.
Yield: 29.6 g (81.5 %) of yellow oil.
2-(E)-Butenedioate (1/1) M.p.: 128 - 130 °C.

| Analysis for the formula $C_{25}H_{35}ClN_2O_5$ (479.0): | | | | |
|---|---|---|---|---|
| Calculated:<br>Found: | C % = 62.68<br>C % = 62.59 | H % = 7.68<br>H % = 7.73 | Cl% = 7.40<br>Cl% = 7.40 | N % = 5.85<br>N % = 5.83. |

UV: $\lambda_{max}$ = 289 nm ($\epsilon$ = 30417).

**Example 5**

**1-Phenyl-6-methyl-3-(E)-[3-(dimethylamino)-propoxyimino]-1-(E)-heptene**

1-Phenyl-6-methyl-1-(E)-hepten-3-one (20.2 g; 0.1 mole) and O-[3-(dimethylamino)-propyl]-hydroxylamine hydrochloride (19.1 g; 0.1 mole) are boiled in a mixture of 200 $cm^3$ of anhydrous ethanol and 75 $cm^3$ of pyridine for 2 hours. The solvent is then removed in vacuo, the residue is rendered alkaline with an aqueous sodium hydroxide solution (pH = 10) at a temperature of 5 °C, the base is extracted with dichloroethane, and the organic phase is dried and evaporated.
Yield: 29.6 g (97.8 %) of yellow oil.
2-(E)-Butenedioate (1/1) M.p.: 108 - 109 °C.

| Analysis for the formula $C_{23}H_{34}N_2O_5$ (418.5): | | | |
|---|---|---|---|
| Calculated:<br>Found: | C % = 66.00<br>C % = 65.87 | H % = 8.19<br>H % = 8.22 | N % = 6.70<br>N % = 6.83. |

UV: $\lambda_{max}$ = 285 nm ($\epsilon$ = 27646).

**Example 6**

**1-Phenyl-3-(E)-[3-(N-piperidinyl)-propoxyimino]-1-(E)-hexene**

One proceeds as specified in Example 1 except that instead of 2-chloro-N,N-diethylethylamine 1-(3-chloroethyl)-piperidine (17.8 g; 0.1 mole) is used.
Yield: 30.2 g (96.2 %) of yellow oil.
2-(E)-Butenedioate (1/1) M.p.: 91 - 93 °C.

| Analysis for the formula $C_{24}H_{34}N_2O_5$ (430.5): | | | |
|---|---|---|---|
| Calculated:<br>Found: | C % = 66.95<br>C % = 66.94 | H % = 7.96<br>H % = 7.96 | N % = 6.51<br>N % = 6.55. |

UV: $\lambda_{max}$ = 289 nm ($\epsilon$ = 21693).

**Example 7**

**1-(4-Chlorophenyl)-3-(E)-[2-(dimethylamino)-ethoxy-imino]-1-(E)-pentene**

A solution of 1-(4-chlorophenyl)-1-(E)-penten-3-one-(E)-oxime (21.0 g; 0.1 mole) in 20 $cm^3$ of dioxane is dropped into a suspension of potassium amide (5.5 g, 0.1 mole) in 100 $cm^3$ of dioxane. When the gas evolution ceases, a benzene (20 $cm^3$) solution of 2-chloro-N,N-dimethylethylamine (11.8 g, 0.11 mole) is added to the suspension. The mixture is reacted at a temperature between 60 °C and 80°C until the starting oxime cannot be detected any more by thin layer chromatography as described in Example 1. The reaction mixture is then washed with water, extracted with 10% by weight aqueous tartaric acid solution, a concentrated aqueous ammonium hydroxide solution is added to it, it is extracted with dichloromethane, dried and evaporated.
Yield: 23.5 g (83.6 %) of yellow oil.
2-(E)-Butenedioate (1/1) M.p.: 160 - 162 °C.

| Analysis for the formula $C_{19}H_{25}ClN_2O_5$ (396.9): | | | | |
|---|---|---|---|---|
| Calculated: | C % = 57.40 | H % = 6.35 | Cl% = 8.93 | N % = 7.06 |
| Found: | C % = 57.30 | H % = 6.38 | Cl% = 8.95 | N % = 7.11. |

UV: $\lambda_{max}$ = 292 nm ($\epsilon$ = 34736).

**Example 8**

**(R,S)-1-(4-Chlorophenyl)-6-methyl-3-(E)-{3-[bis(1-methylethyl)-amino]-2-hydroxypropoxyimino}-1-(E)-heptene**

1-(4-Chlorophenyl)-6-methyl-1-(E)-hepten-3-one-(E)-oxime (25.2 g; 0.1 mole) is converted into a salt with the aid of sodium hydride (4.8 g; 0.1 mole) in a mixture of 30 $cm^3$ of dimethylformamide and 20 $cm^3$ of benzene, at a temperature of 25°C. When the gas evolution ceases, 1-chloro-2,3-epoxypropane (10.2 g; 0.11 mole) is added to the suspension and the mixture is reacted at a temperature of 55 °C to 60 °C for 5 hours. Then it is washed with water, and the organic phase is dried and evaporated. To the residual brown oil (26.1 g) 100 $cm^3$ of anhydrous ethanol and 20.2 g (0.2 mole) of N,N-bis-[1-(methyl)-ethyl]-amineare added and the mixture is boiled for 5 hours. The solution is evaporated and the product is precipitated as specified in Example 1.
Yield: 28.4 g (69.5 %) of brownish yellow oil.
2-(E)-Butenedioate M.p.: 88 - 90 °C.

| Analysis for the formula $C_{27}H_{41}ClN_2O_6$ (525.0): | | | | |
|---|---|---|---|---|
| Calculated: | C % = 61.76 | H % = 7.87 | Cl% = 6.75 | N % = 5.34 |
| Found: | C % = 61.56 | H % = 7.93 | Cl% = 6.63 | N % = 5.42. |

UV: $\lambda_{max}$ = 289 nm ($\epsilon$ = 28387).

**Example 9**

**1-(4-Chlorophenyl)-6-methyl-3-(E)-[3-(N-piperidinyl)-propoxyimino]-1-(E)-heptene**

One proceeds as specified in Example 6 except that 1-(4-chlorophenyl)-6-methyl-1-(E)-hepten-3-one-(E)-oxime (25.1 g; 0.1 mole) is used as starting substance.
Yield: 34.4 g (91.2 %) of yellow oil.
2-(E)-Butenedioate (1/1) M.p.: 121 - 124 °C.

| Analysis for the formula $C_{26}H_{37}ClN_2O_5$ (493.0): | | | | |
|---|---|---|---|---|
| Calculated: | C % = 63.34 | H % = 7.57 | Cl% = 7.19 | N % = 5.69 |
| Found: | C % = 63.32 | H % = 7.51 | Cl% = 7.24 | N % = 5.72. |

UV: $\lambda_{max}$ = 290 nm ($\epsilon$ = 28269).

**Example 10**

**1-Phenyl-5-methyl-3-(E)-[2-(N-piperidinyl)-ethoxy-imino]-1-(E)-hexene**

One proceeds as specified in Example 3 except that 1-phenyl-5-methyl-1-hexen-3-one-(E)-oxime (20.3 g, 0.1 mole) is used as starting substance.
Yield: 22.8 g (72.4 %) of yellow oil.
2-(E)-Butenedioate (1/1) M.p.: 140 - 143 °C.

| Analysis for the formula $C_{24}H_{34}N_2O_5$ (430.5): | | | |
|---|---|---|---|
| Calculated: | C % = 66.95 | H % = 7.96 | N % = 6.51 |
| Found: | C % = 67.03 | H % = 7.89 | N % = 6.46. |

UV: $\lambda_{max}$ = 287 nm ($\epsilon$ = 24627).

**Example 11**

**1-Phenyl-3-(E)-[2-(N-morpholinylothoxyimino]-1-(E)-pentene**

One proceeds as specified in Example 1 except that 1-phenyl-1-(E)-penten-3-one-(E)-oxime (17.5 g; 0.1 mole) is used as starting oxime and N-(3-chloroethyl)-morpholine (18.0 g; 0.11 mole) is applied instead of 2-chloro-N,N-diethylethylamine.
Yield: 25.0 g (86.8 %) of yellow oil.
2-(E)-Butenedioate (1/1) M.p.: 115 - 117 °C.

| Analysis for the formula $C_{21}H_{28}N_2O_6$ (404.4): | | | |
|---|---|---|---|
| Calculated: | C % = 62.36 | H % = 6.98 | N % = 6.93 |
| Found: | C % = 62.43 | H % = 6.87 | N % = 6.95. |

UV: $\lambda_{max}$ = 286 nm ($\epsilon$ = 27290).

**Example 12**

**1-(4-Chlorophenyl)-3-(E)-[2-(N-piperidinyl)-ethoxyimino]-1-(E)-hexene**

One proceeds as specified in Example 3 except that 1-(4-chlorophenyl)-1-(E)-hexen-3-one-(E)-oxime (22.4 g; 0.1 mole) is used as starting oxime.
Yield: 28.8 g (86.0 %) of yellow oil.
2-(E)-Butenedioate (1/1) M.p.: 161 - 163 °C.

| Analysis for the formula $C_{23}H_{31}ClN_2O_5$ (451.0): | | | | |
|---|---|---|---|---|
| Calculated: | C % = 61.25 | H % = 6.93 | Cl% = 7.86 | N % = 6.21 |
| Found: | C % = 61.33 | H % = 6.99 | Cl% = 7.75 | N % = 6.31. |

UV: $\lambda_{max}$ = 289 nm ($\epsilon$ = 32150).

### Example 13

**1-(4-Chlorophenyl)-6-methyl-3-(E)-[3-(dimethylamino)-propoxyimino]-1-(E)-heptene**

1-(4-Chlorophenyl)-6-methyl-1-(E)-hepten-3-one-(E)-oxime (0.1 mole; 25.1 g) is reacted with 3-chloro-N,N-dimethylpropylamine in a mixture of 50 % by weight of aqueous potassium hydroxide solution and 10 g of dimethyl sulfoxide at a temperature between 50 °C and 60 °C for 3 hours. The product is extracted and purified by acidic-alkaline precipitation.
Yield: 30.8 g (91.4 %) of yellow oil.
2-(E)-Butenedioate (1/1) M.p.: 115 - 120 °C.

| Analysis for the formula $C_{23}H_{33}ClN_2O_5$ (453.0): | | | | |
|---|---|---|---|---|
| Calculated: | C % = 60.98 | H % = 7.31 | Cl% = 7.83 | N % = 6.18 |
| Found: | C % = 61.01 | H % = 7.28 | Cl% = 7.86 | N % = 6.21. |

UV: $\lambda_{max}$ = 291 nm ($\epsilon$ = 31252).

### Example 14

**1-(Phenyl-3-(E)-[2-(dimethylamino)-ethoxyimino]-1-(E)-pentene**

One proceeds as specified in Example 7 except that 1-phenyl-1-(E)-penten-3-one-(E)-oxime (17.5 g; 0.1 mole) is used as starting oxime.
Yield: 17.0 g (68.9 %) of yellow oil.
2-(E)-Butenedioate (1/1) M.p.: 112 - 114 °C.

| Analysis for the formula $C_{19}H_{26}N_2O_5$ (362.4): | | | |
|---|---|---|---|
| Calculated: | C % = 62.97 | H % = 7.23 | N % = 7.73 |
| Found: | C % = 63.05 | H % = 7.12 | N % = 7.68. |

UV: $\lambda_{max}$ = 284 nm ($\epsilon$ = 26774).

### Example 15

**1-(4-Chlorophenyl)-6-methyl-3-(E)-[2'-(dimethylamino)-ethoxyimino]-1-(E)-heptene**

One proceeds as specified in Example 4 except that 2-chloro-N,N-dimethylethylamine (11.9 g; 0.11 mole) is used as alkylating agent.
Yield: 23.7 g (73.4 %) of yellow oil.
2-(E)-Butenedioate (1/1) M.p.: 149 - 151 °C.

| Analysis for the formula $C_{22}H_{31}ClN_2O_5$ (438.9): | | | | |
|---|---|---|---|---|
| Calculated: | C % = 60.20 | H % = 7.12 | Cl% = 8.08 | N % = 6.38 |
| Found: | C % = 59.94 | H % = 7.06 | Cl% = 8.02 | N % = 6.31. |

UV: $\lambda_{max}$ = 289 nm ($\epsilon$ = 28671).

### Example 16

**1-Phenyl-3-(E)-[3-(dimethylamino)-propoxyimino]-1-(E)-pentene**

One proceeds as specified in Example 11 except that 3-chloro-N,N-dimethylpropylamine (13.4 g; 0.11 mole) is used as alkylating agent.
Yield: 20.7 g (79.6 %) of yellow oil.

2-(Z)-Butenedioate (1/1) M.p.: 69 - 71 °C.

| Analysis for the formula $C_{20}H_{28}N_2O_5$ (376.3): | | | |
|---|---|---|---|
| Calculated: | C % = 63.81 | H % = 7.50 | N % = 7.44 |
| Found: | C % = 63.72 | H % = 7.53 | N % = 7.45. |

UV: $\lambda_{max}$ = 286 nm ($\epsilon$ = 27519).

**Example 17**

**1-Phenyl-5-methyl-3-(E)-[2-(dimethylamino)-ethoxyimino]-1-(E)-hexene**

One proceeds as specified in Example 7 except that 1-phenyl-5-methyl-1-(E)-hexen-3-one-(E)-oxime (20.3 g; 0.1 mole) is used as starting oxime.
Yield: 20.1 g (73.2 %) of yellow oil.
2-(E)-Butenedioate (1/1) M.p.: 133 - 135 °C.

| Analysis for the formula $C_{21}H_{30}N_2O_5$ (390.5): | | | |
|---|---|---|---|
| Calculated: | C % = 64.59 | H % = 7.74 | N % = 7.17 |
| Found: | C % = 64.18 | H % = 7.82 | N % = 7.21. |

UV: $\lambda_{max}$ = 286 nm ($\epsilon$ = 26696).

**Example 18**

**(R,S)-1-Phenyl-3-(E)-[3-(dimethylamino)-2-methylpropoxyimino]-1-(E)-pentene**

One proceeds as specified in Example 16 except that 3-chloro-2,N,N-trimethylpropylamine (14.9 g; 0.11 mole) is used for the alkylation.
Yield: 23.3 g (84.9 %) of yellow oil.
2-(Z)-Butenedioate (1/1) M.p.: 88 - 92 °C.

| Analysis for the formula $C_{21}H_{30}N_2O_5$ (390.5): | | | |
|---|---|---|---|
| Calculated: | C % = 64.59 | H % = 7.74 | N % = 7.17 |
| Found: | C % = 64.63 | H % = 7.80 | N % = 7.19. |

UV: $\lambda_{max}$ = 277 nm ($\epsilon$ = 28176).

**Example 19**

**(R,S)-1-(4-Chlorophenyl)-3-(E)-[3-(dimethylamino)-2-(methylpropoxyimino]-1-(E)-pentene**

One proceeds as specified in Example 7 except that 3-chloro-2,N,N-trimethylpropylamine (14.9 g; 0.1 mole) is used as alkylating agent.
Yield: 23.7 g (76.8 %) of yellow viscous oil.
2-(E)-Butenedioate (1/1) M.p.: 122 - 124 °C.

| Analysis for the formula $C_{21}H_{29}ClN_2O_5$ (424.9): | | | | |
|---|---|---|---|---|
| Calculated: | C % = 59.36 | H % = 6.87 | Cl% = 8.35 | N % = 6.59 |
| Found: | C % = 59.17 | H % = 6.94 | Cl% = 8.23 | N % = 6.66. |

UV: $\lambda_{max}$ = 291 nm ($\epsilon$ = 33817).

**Example 20**

**1-(3-Methoxyphenyl)-3-(E)-[2-bis(1-methylethyl)-amino-ethoxyimino]-1-(E)-hexene**

One proceeds as specified in Example 1 except that 1-(3-methoxyphenyl)-1-(E)-hexen-3-one-(E)-oxime (21.9 g; 0.1 mole) is used as starting oxime and N-(2-chloroethyl)-N-(1-methylethyl)-2-propylamine (18.0 g; 0.11 mole) is applied as alkylating agent.
Yield: 30.4 g (87.8 %) of yellow oil.
2-(E)-Butenedioate (1/1) M.p.: 130 - 132 °C.

| Analysis for the formula $C_{25}H_{38}N_2O_6$ (462.6): | | | |
|---|---|---|---|
| Calculated: | C % = 64.91 | H % = 8.28 | N % = 6.06 |
| Found: | C % = 64.78 | H % = 8.25 | N % = 6.16. |

UV: $\lambda_{max}$ = 280 and 322 nm ($\epsilon$ = 18863 and 16048).

**Example 21**

**1-Phenyl-3-(E)-[3-(dimethylamino)-propoxyimino]-1-(E)-hexene**

One proceds as specified in Example 1 except that 3-chloro-N,N-dimethylpropylamine (13.4 g; 0.1 mole) is used as alkylating agent.
Yield: 26.6 g (96.8 %) of yellowish brown oil.
2-(E)-Butenedioate (1/1) M.p.: 103 - 106 °C.

| Analysis for the formula $C_{21}H_{30}N_2O_5$ (390.5): | | | |
|---|---|---|---|
| Calculated: | C % = 64.59 | H % = 7.74 | N % = 7.18 |
| Found: | C % = 64.50 | H % = 7.82 | N % = 7.16. |

UV: $\lambda_{max}$ = 287 nm ($\epsilon$ = 29527).

**Example 22**

**1-(2-Methoxyphenyl)-3-(E)-[2-(dimethylamino)-ethoxy-imino]-1-(E)-hexene**

One proceeds as specified in Example 1 except that 2-chloro-N,N-dimethylethylamine (11.8 g; 0.1 mole) is used as alkylating agent and 1-(2-methoxyphenyl)-1-(E)-hexen-3-one-(E)-oxime (21.9 g; 0,1 mole) is applied as starting oxime.
Yield: 25.3 g (87.3 %) of yellow oil.
2-(E)-Butenedioate (1/1) M.p.: 115 - 117 °C.

| Analysis for the formula $C_{21}H_{30}N_2O_6$ (406.4): | | | |
|---|---|---|---|
| Calculated: | C % = 62.06 | H % = 7.43 | N % = 6.89 |
| Found: | C % = 62.13 | H % = 7.52 | N % = 6.91. |

UV: $\lambda_{max}$ = 280 and 318 nm ($\epsilon$ = 18014 and 14705).

**Example 23**

**1-(4-Methoxyphenyl)-3-(E)-[2-(diethylamino)-ethoxyimino]-1-(E)-pentene**

One proceeds as specified in Example 1 except that 1-(4-methoxyphenyl)-1-(E)-penten-3-one-(E)-oxime (20.5 g; 0.1 mole) is used as starting oxime.
Yield: 30.4 g (83.4 %) of yellow oil.

2-(E)-Butenedioate (1/1) M.p.: 114 - 116 °C.

| Analysis for the formula $C_{22}H_{32}N_2O_6$ (420.5): | | | |
|---|---|---|---|
| Calculated: | C % = 62.83 | H % = 7.67 | N % = 6.66 |
| Found: | C % = 62.65 | H % = 7.65 | N % = 6.68. |

UV: $\lambda_{max}$ = 308 nm ($\epsilon$ = 23548).

**Example 24**

**1-(2-Methoxyphenyl)-3-(E)-[3-(dimethylamino)-2-methylpropoxyimino]-1-(E)-pentene**

One proceeds as specified in Example 18 except that 1-(2-methoxyphenyl)-1-(E)-penten-3-one-(E)-oxime (20.5 g; 0.2 mole) is used as starting oxime.
Yield: 23.6 g (77.4 %) of yellow oil.
2-(E)-Butenedioate (1/1) M.p.: 130 - 132 °C.

| Analysis for the formula $C_{22}H_{32}N_2O_6$ (420.5): | | | |
|---|---|---|---|
| Calculated: | C % = 62.83 | H % = 7.67 | N % = 6.66 |
| Found: | C % = 62.57 | H % = 7.58 | N % = 6.76. |

UV: $\lambda_{max}$ = 278 and 314 nm ($\epsilon$ = 14998 and 12394).

**Example 25**

**1-(4-Methoxyphenyl)-3-(E)-[2-(dimethylamino)-ethoxyimino]-1-(Z,E)-pentene**

One proceeds as specified in Example 1 except that 1-(4-methoxyphenyl)-1-(E)-penten-3-one-(E)-oxime (20.5 g; 0.1 mole) is used as starting oxime and 2-chloro-N,N-dimethylethylamine (11.89 g; 0.11 mole) is applied as alkylating agent.
Yield: 17.5 g (63.4 %) of yellow oil.
2-(E)-Butenedioate (1/1) M.p.: 138 - 139 °C.

| Analysis for the formula $C_{20}H_{28}N_2O_6$ (392.4): | | | |
|---|---|---|---|
| Calculated: | C % = 61.21 | H % = 7.19 | N % = 7.14 |
| Found: | C % = 61.33 | H % = 7.11 | N % = 7.08. |

UV: $\lambda_{max}$ = 297 nm ($\epsilon$ = 23469).
Isomeric ratio (Z) : (E) = 4:3.

**Example 26**

**1-(4-chlorophenyl)-3-(E)-[2-(diethylamino)-ethoxyimino]-1-(E)-pentene**

One proceeds as specified in Example 7 except that 2-chloro-N,N-diethylethylamine (14.9 g; 0.11 mole) is used as alkylating agent.
Yield: 25.2 g (81.73 %) of yellow oil.
2-(E)-Butenedioate (1/1) M.p.: 162 - 165 °C.

| Analysis for the formula $C_{21}H_{29}ClN_2O_5$ (424.9): | | | |
|---|---|---|---|
| Calculated: | C % = 59.36 | H % = 6.88 | N % = 6.59 |
| Found: | C % = 58.99 | H % = 6.83 | N % = 6.63. |

UV: $\lambda_{max}$ = 288 nm ($\epsilon$ = 34000).

**Example 27**

**1-(4-Chlorophenyl)-3-(E)-[2-(diethylamino)-ethoxyimino]-1-(E)-hexene**

One proceeds according to Example 1 except that 1-(4-chlorophenyl)-1-(E)-hexen-3-one-(E)-oxime (22.0 g; 0.1 mole) is used as starting oxime.
Yield: 28.0 g (86.8 %) of yellow oil.
2-(E)-Butenedioate (1/1) M.p.: 125 - 127 °C.

| Analysis for the formula $C_{22}H_{31}ClN_2O_5$ (438.9): | | | | |
|---|---|---|---|---|
| Calculated: | C % = 60.26 | H % = 7.13 | Cl% = 8.09 | N % = 6.39 |
| Found: | C % = 60.27 | H % = 7.21 | Cl% = 8.11 | N % = 6.43. |

UV: $\lambda_{max}$ = 280 nm ($\epsilon$ = 34589).

**Example 28**

**1-(4-Chlorophenyl)-3-(E)-[2-(dimethylamino)-ethoxyimino]-1-(E)-hexene**

One proceeds as specified in Example 1 except that 1-(4-chlorophenyl)-1-(E)-hexen-3-one-(E)-oxime (22.0 g; 0.1 mole) is used as starting oxime and 2-chloro-N,N-dimethylethylamine (11.8 g; 0.11 mole) is applied as alkylating agent.
Yield: 23.4 g (79.3 %) of yellow oil.
2-(E)-Butenedioate (1/1) M.p.: 132 - 135 °C.

| Analysis for the formula $C_{20}H_{21}ClN_2O_5$ (410.9): | | | | |
|---|---|---|---|---|
| Calculated: | C % = 58.46 | H % = 6.62 | Cl% = 8.63 | N % = 6.82 |
| Found: | C % = 58.58 | H % = 6.67 | Cl% = 8.55 | N % = 6.79. |

UV: $\lambda_{max}$ = 280 nm ($\epsilon$ = 31474).

**Example 29**

**(R,S)-1-Phenyl-3-(E)-[3-(dimethylamino)-2-methyl-propoxyimino]-1-(E)-butene**

A mixture of 1-phenyl-1-(E)-buten-3-one-(E)-oxime (16.2 g; 0.1 mole), 4,4 g (0.11 mole) of sodium hydroxide, 200 $cm^3$ of toluene and 20 $cm^3$ of dimethyl sulfoxide is boiled in a flask equipped with a Marcusson trap until the distillation of water ceases. The sodium salt thus obtained is subjected to condensation with 3-chloro-2,N,N-trimethylpropylamine (16.2 g; 0.12 mole) at 80 °C until the starting oxime cannot be detected any more in the reaction mixture in the way as specified in Example 1. The product is purified by acidic-alkaline precipitation.
Yield: 22.7 g (86.7 %) of yellow oil.
2-(E)-Butenedioate (1/1) M.p.: 115 - 117 °C.

| Analysis for the formula $C_{20}H_{29}N_2O_5$: | | | |
|---|---|---|---|
| Calculated: | C % = 63.64 | H % = 7.68 | N % = 7.43 |
| Found: | C % = 63.58 | H % = 7.51 | N % = 7.55. |

UV: $\lambda_{max}$ = 285 nm ($\epsilon$ = 32603).

**Example 30**

**1-Phenyl-3-(E)-[3-(4-methyl-1-piperazinyl)-propoxyimino]-1-(E)-butene**

One proceeds as specified in Example 29 except that instead of 3-chloro-2,N,N-trimethylpropylamine 1-chloropropyl-4-methylpiperazine (21.1 g; 0.12 mole) is used.
Yield: 27.7 g (91.7 %) of yellow oil.
2-(E)-Butenedioate (1/2) M.p.: 208 - 210 °C.

| Analysis for the formula $C_{26}H_{35}N_3O_9$ (533.6): | | | |
|---|---|---|---|
| Calculated: | C % = 58.53 | H % = 6.67 | N % = 7.87 |
| Found: | C % = 58.61 | H % = 6.71 | N % = 7.83. |

UV: $\lambda_{max}$ = 285 nm ($\epsilon$ = 29983).

**Example 31**

**1-Phenyl-3-(E)-[3-(dimethylamino)-propoxyimino]-1-(E)-butene**

One proceeds as specified in Example 13 except that instead of 1-(4-chlorophenyl)-6-methyl-1-(E)-hepten-3-one-(E)-oxime 16.2 g (0.1 mole) of 1-Phenyl-1-(E)-buten-3-one-(E)-oxime are used.
Yield: 23.3 g (94.2 %) of yellow oil.
2-(E)-Butenedioate (1/1) M.p.: 117 - 119 °C.

| Analysis for the formula $C_{19}H_{27}N_2O_5$ (363.44): | | | |
|---|---|---|---|
| Calculated: | C % = 64.16 | H % = 7.66 | N % = 7.86 |
| Found: | C % = 63.98 | H % = 7.17 | N % = 7.78. |

UV: $\lambda_{max}$ = 285 nm ($\epsilon$ = 31855).

**Example 32**

**1-Phenyl-6-methyl-3-(E)-{2-[bis-1-(methylethyl)-amino]-ethoxyimino}-1-(E)-heptene**

One proceeds as specified in Example 1 except that instead of 1-phenyl-1-(E)-hexen-3-one-(E)-oxime 1-phenyl-6-methyl-1-(E)-hepten-3-one-(E)-oxime (23.1 g; 0.1 mole) and instead of 2-chloro-N,N-diethylethylamine 18.01 g (0.11 mole) of N-(2-chloroethyl)-N-(1-methylethyl)-2-propylamine is used.
Yield: 31.1 g (90.2 %) of yellow viscous oil.
2-(E)-Butenedioate (1/1) M.p.: 104 - 106 °C.

| Analysis for the formula $C_{25}H_{40}N_2O_5$ (460.62): | | | |
|---|---|---|---|
| Calculated: | C % = 67.79 | H % = 8.75 | N % = 6.08 |
| Found: | C % = 67.85 | H % = 8.48 | N % = 6.19. |

UV: $\lambda_{max}$ = 288 nm ($\epsilon$ = 29969).

**Example 33**

**1-Phenyl-6-methyl-3-(E)-[3-(4-methyl-1-piperazinyl)-propoxyimino)-1-(E)-heptene**

One proceeds as specified in Example 32 except that instead of N-(2-chloroethyl)-N-(1-methylethyl)-2-propylamine 1-chloropropyl-4-methylpiperazine (21.1 g; 0.12 mole) is used.
Yield: 32.8 g (91.9 %) of yellow viscous oil.
2-(E)-Butenedioate (1/2) M.p.: 206 - 211 °C.

| Analysis for the formula $C_{30}H_{43}N_3O_9$ (589.70): | | | |
|---|---|---|---|
| Calculated:<br>Found: | C % = 61.10<br>C % = 61.28 | H % = 7.32<br>H % = 7.36 | N % = 7.18<br>N % = 7.12. |

UV: $\lambda_{max}$ = 287 nm ($\epsilon$ = 31791).

**Example 34**

**1-(4-Chlorophenyl)-6-methyl-3-(E)-[3-(hexahydro-1H-azepinyl)-propoxyimino]-1-(E)-heptene**

One proceeds as specified in Example 1 except that 1-(4-chlorophenyl)-6-methyl-1-(E)-hepten-3-one-(E)-oxime (25.2 g; 0.1 mole) is used as oxime and instead of 2-chloro-N,N-diethylethylamine 19.1 g (0.11 mole) of N-(3-chloropropyl)-hexahydro-1H-azepine are applied.
Yield: 35.1 g (93.0 %) of yellow oil.
2-(E)-Butenedioate (1/1) M.p.: 116 - 117 °C.

| Analysis for the formula $C_{27}H_{39}ClN_2O_5$ (493.0): | | | | |
|---|---|---|---|---|
| Calculated:<br>Found: | C % = 63.95<br>C % = 63.84 | H % = 7.75<br>H % = 7.79 | Cl% = 5.52<br>Cl% = 5.61 | N % = 6.99<br>N % = 7.00. |

UV: $\lambda_{max}$ = 290 nm ($\epsilon$ = 29866).

**Example 35**

**(R,S)-1-(4-Chlorophenyl)-6-methyl-3-(E)-[3-(cyclohexylamino)-2-hydroxypropoxyimino]-1-(E)-heptene**

One proceeds as specified in Example 8 except that after the etherification the epoxy group is reacted with cyclohexylamine instead of N,N-bis-[1-(methyl)-ethyl]-amine.
Yield: 34.7 g (85.2 %) of white crystals. M.p.: 99 °C.
2-(E)-Butenedioate (2/1) M.p.: 155 °C.

| Analysis for the formula $C_{25}H_{37}ClN_2O_4$ (465.0): | | | | |
|---|---|---|---|---|
| Calculated:<br>Found: | C % = 64.57<br>C % = 64.48 | H % = 8.02<br>H % = 8.04 | Cl% = 6.03<br>Cl% = 6.12 | N % = 7.63<br>N % = 7.61. |

UV: $\lambda_{max}$ = 290 nm ($\epsilon$ = 31782).

**Example 36**

**(R,S)-1-(4-Chlorophenyl)-6-methyl-3-(E)-[2-hydroxy-3-(1-piperidinyl)-propoxyimino]-1-(E)-heptene**

One proceeds as specified in Example 8 except that the epoxy group is reacted with piperidine.
Yield: 27.2 g (69.4 %) of yellow viscous oil.
2-(E)-Butenedioate (1/1) M.p.: 101 to 103 °C.

| Analysis for the formula $C_{26}H_{37}ClN_2O_6$ (509.0): | | | | |
|---|---|---|---|---|
| Calculated: | C % = 61.34 | H % = 7.32 | Cl% = 5.50 | N % = 6.96 |
| Found: | C % = 61.25 | H % = 7.28 | Cl% = 5.44 | N % = 6.89. |

UV: $\lambda_{max}$ = 291 nm ($\epsilon$ = 30739).

**Example 37**

**1-(4-Fluorophenyl)-6-methyl-3-(E)-[3-(N-piperidinyl)propoxyimino)-1-(E)-heptene**

One proceeds as specified in Example 1 except that 23.5 g (0.1 mole) of 1-(4-fluorophenyl)-6-methyl-1-(E)-hepten-3-one-(E)-oxime are used as oxime and 1-(3-chloropropyl)-piperidine (17.5 g; 0.11 mole) is applied as alkylating agent.
Yield: 29.4 g (81.4 %) of viscous yellow oil.
2-(E)-Butenedioate (1/1) M.p.: 127 to 128 °C.

| Analysis for the formula $C_{26}H_{37}FN_2O_5$ (476.6): | | | | |
|---|---|---|---|---|
| Calculated: | C % = 65.38 | H % = 7.81 | N % = 5.87 | F % = 3.98 |
| Found: | C % = 65.44 | H % = 7.80 | N % = 5.93 | F % = 3.89. |

UV: $\lambda_{max}$ = 283 nm ($\epsilon$ = 27747).

**Example 38**

**1-(4-Chlorophenyl)-6-methyl-3-(E)-[3-(N-morpholinyl)-propoxyimino]-1-(E)-heptene**

One proceeds as specified in Example 1 except that 25.2 g (0.1 mole) of 1-(4-chlorophenyl)-6-methyl-1-(E)-hepten-3-one-(E)-oxime are used as oxime and 1-(3-chloropropyl)-morpholine (18.0 g; 0.11 mole) is applied for the alkylation.
Yield: 33.5 g (88.3 %) of yellow viscous oil.
2-(E)-Butenedioate (1/1) M.p.: 130 to 135 °C.

| Analysis for the formula $C_{25}H_{35}ClN_2O_6$ (495.0): | | | | |
|---|---|---|---|---|
| Calculated: | C % = 60.65 | H % = 7.13 | N % = 5.66 | Cl% = 7.16 |
| Found: | C % = 60.48 | H % = 7.11 | N % = 5.70 | Cl% = 7.05. |

UV: $\lambda_{max}$ = 290 nm ($\epsilon$ = 28054).

**Example 39**

**1-(4-Chlorophenyl)-6-methyl-3-(Z)-[2-(2-N-methylpyrrolidinyl)-ethoxyimino]-1-(E)-heptene**

One proceeds as specified in Example 38 except that 2-(2-chloroethyl)-N-methylpyrrolidine (16.2 g; 0.11 mole) is used for the alkylation.
Yield: 13.2 g (36.5 %) of yellow oil.
2-(Z)-Butenedioate M.p.: 142 to 145 °C.

| Analysis for the formula $C_{25}H_{35}ClN_2O_5$ (479.0): | | | | |
|---|---|---|---|---|
| Calculated: | C % = 62.68 | H % = 7.37 | N % = 5.85 | Cl% = 7.40 |
| Found: | C % = 62.74 | H % = 7.49 | N % = 5.78 | Cl% = 7.42. |

UV: $\lambda_{max}$ = 290 nm ($\epsilon$ = 32050).

**Example 40**

**1-(4-Chlorophenyl)-6-methyl-3-(E)-[3-(N-pyrrolidinyl)-propoxyimino]-1-(E)-heptene**

One proceeds as specified in Example 38 except that 1-(3-chloropropyl)-pyrrolidine (16.0 g; 0.11 mole) is used for the alkylation.
Yield: 29.2 g (80.5 %) of yellow viscous oil.
2-(E)-Butenedioate (1/1) M.p.: 115 to 118 °C.

| Analysis for the formula $C_{25}H_{35}ClN_2O_5$ (479.0): | | | | |
|---|---|---|---|---|
| Calculated:<br>Found: | C % = 62.68<br>C % = 62.77 | H % = 7.37<br>H % = 7.43 | N % = 5.85<br>N % = 5.82 | Cl% = 7.40<br>Cl% = 7.38. |

UV: $\lambda_{max}$ = 290 nm ($\epsilon$ = 31034).

**Example 41**

**1-(4-Bromophenyl)-6-methyl-3-(E)-[(3-N-piperidinyl)-propoxyimino]-1-(E)-heptene**

One proceeds as specified in Example 6 except that 1-(4-bromophenyl)-6-methyl-1-(E)-hepten-3-one-(E)-oxime (28.6 g; 0.1 mole) is used as oxime.
Yield: 39.3 g (93.2 %) of yellow oil.
2-(E)-Butenedioate (1/1) M.p.: 122 to 124 °C.

| Analysis for the formula $C_{26}H_{37}BrN_2O_5$ (537.5): | | | | |
|---|---|---|---|---|
| Calculated:<br>Found: | C % = 58.09<br>C % = 57.75 | H % = 6.94<br>H % = 7.00 | N % = 5.21<br>N % = 5.17 | Br% = 14.87<br>Br% = 14.62. |

UV: $\lambda_{max}$ = 282 nm ($\epsilon$ = 29780).

**Example 42**

**1-(3,4-Dichlorophenyl)-6-methyl-3-(E)-[(3-N-piperidinyl)-propoxyimino]-1-(E)-heptene**

One proceeds according to Example 37 except that 1-(3,4-dichlorophenyl)-1-(E)-hepten-3-one-(E)-oxime (28.5 g, 0.1 mole) is used as oxime.
Yield: 32.2 g (78.2 %) of viscous oil.
2-(E)-Butenedioate (1/1) M.p.: 138 to 139 °C

| Analysis for the formula $C_{26}H_{36}Cl_2N_2O_5$ (527.49): | | | | |
|---|---|---|---|---|
| Calculated:<br>Found: | C % = 59.30<br>C % = 59.30 | H % = 6.90<br>H % = 6.90 | Cl% = 13.44<br>Cl% = 13.46 | N % = 5.37<br>N % = 5.37. |

UV: $\lambda_{max1}$ = 300.7 nm ($\epsilon_1$: 32727).

**Example 43**

**1-(3,4-Methylenedioxyphenyl)-6-methyl-3-(E)-[(3-N-piperidinyl)-propoxyimino)-1-(E)-heptene**

One proceeds as specified in Example 37 except that 1-(3,4-methylenedioxyphenyl)-6-methyl-1-(E)-hepten-3-one-(E)-oxime (26.0 g, 0.1 mole) is used as oxime.
Yield: 33.7 g (87.2 %) of viscous oil.
2-(E)-Butenedioate (1/1) M.p.: 139 to 141.5 °C.

| Analysis for the formula $C_{27}H_{38}N_2O_7$ (502.61): | | | |
|---|---|---|---|
| Calculated:<br>Found: | C % = 64.45<br>C % = 64.45 | H % = 7.86<br>H % = 7.83 | N % = 5.66<br>N % = 5.69. |

UV: $\lambda_{max1}$ = 329 nm ($\epsilon_1$: 21760).
$\lambda_{max2}$ = 300 nm ($\epsilon_2$: 20226).

**Example 44**

**1-(4-Chlorophenyl)-6-methyl-3-(E)-[2-(diethylamino)-ethoxyimino]-1-(E)-heptene**

One proceeds as specified in Example 4 except that the alkylation is carried out with 2-chloro-N,N-diethyl-ethyl amine (14.9 g, 0.11 mole).
Yield: 27.7 g (78.9 %) of yellow viscous oil.
2-(E)-Butenedioate (1/1) M.p.: 112 to 113 °C.

| Analysis for the formula $C_{24}H_{35}ClN_2O_5$ (466.98): | | | | |
|---|---|---|---|---|
| Calculated:<br>Found: | C % = 61.72<br>C % = 61.52 | H % = 7.55<br>H % = 7.63 | Cl% = 7.59<br>Cl% = 7.51 | N % = 6.01<br>N % = 6.02. |

UV: $\lambda_{max1}$ = 289 nm ($\epsilon_1$: 31905).

**Example 45**

**(R,S)-1-(4-Chlorophenyl)-6-methyl-3-(E)-[(3-N-pyrrolidinyl-2-hydroxy)-propoxyimino]-1-(E)-heptene**

One proceeds as specified in Example 8 except that instead of N,N-bis-[1-(methyl)-ethyl]-amine pyrrolidine (14.2 g, 0.2 mole) is used for the opening of the oxirane ring.
Yield: 20.2 g (62.8 %) of yellow viscous oil.
2-(E)-Butenedioate (2/1) M.p.: 132 to 133 °C.

| Analysis for the formula $C_{23}H_{33}ClN_2O_4$ (436.98): | | | | |
|---|---|---|---|---|
| Calculated:<br>Found: | C % = 63.21<br>C % = 63.26 | H % = 7.61<br>H % = 7.52 | Cl% = 8.11<br>Cl% = 8.02 | N % = 6.41<br>N % = 6.51. |

UV: $\lambda_{max1}$ = 290 nm ($\epsilon_1$: 31091).

**Example 46**

**(R,S)-1-(4-Chlorophenyl)-6-methyl-3-(E)-[(3-dimethylamino-2-hydroxy)-propoxyimino]-1-(E)-heptene**

One proceeds as specified in Example 8 except that dimethylamine (13.5 g, 0.3 mole) is used for the opening of the oxirane ring.
Yield: 29.1 g (82.4 %) of yellow oil.
2-(E)-Butenedioate (1/1) M.p.: 116 to 117 °C.

| Analysis for the formula $C_{23}H_{33}ClN_2O_6$ (468.98): | | | | |
|---|---|---|---|---|
| Calculated:<br>Found: | C % = 58.90<br>C % = 58.97 | H % = 7.09<br>H % = 7.12 | Cl% = 7.56<br>Cl% = 7.53 | N % = 5.97<br>N % = 5.82. |

UV: $\lambda_{max1}$ = 292 nm ($\epsilon_1$: 32755).

**Example 47**

**(R,S)-1-(4-Methoxyphenyl)-6-methyl-3-(E)-[(3-N-piperidinyl-2-hydroxy)-propoxyimino)-1-(E)-heptene**

One proceeds as specified in Example 8 except that 1-(4-methoxyphenyl)-6-methyl-1-(E)-hepten-3-one-(E)-oxime (24.7 g, 0.1 mole) is used as oxime and piperidine (17.0 g, 0.2 mole) is applied for the opening of the oxirane ring.
Yield: 34.8 g (89.6 %) of yellow oil.
2-(E)-Butenedioate (1/1) M.p.: 129 to 131 °C.

| Analysis for the formula $C_{27}H_{40}N_2O_7$ (504.63): | | | |
|---|---|---|---|
| Calculated: | C % = 64.26 | H % = 7.99 | N % = 5.55 |
| Found: | C % = 64.30 | H % = 7.95 | N % = 5.56. |

UV: $\lambda_{max1}$ = 296 nm $\epsilon_1$: (28926).

**Example 48**

**(R,S)-1-(3,4-Dichlorophenyl)-6-methyl-3-(E)-[(3-N-pyrrolidinyl-2-hydroxy)-propoxyimino]-1-(E)-heptene**

One proceeds as specified in Example 45 except that 1-(3,4-dichlorophenyl)-6-methyl-1-(E)-hepten-3-one-(E)-oxime (28.6 g, 0.1 mole) is used.
Yield: 34.6 g (83.8 %) of yellow oil.
2-(E)-Butenedioate (1/1) M.p.: 110 to 111 °C.

| Analysis for the formula $C_{25}H_{34}Cl_2N_2O_6$ (529.47): | | | | |
|---|---|---|---|---|
| Calculated: | C % = 56.72 | H % = 6.47 | Cl% = 13.39 | N % = 5.29 |
| Found: | C % = 56.50 | H % = 6.53 | Cl% = 13.22 | N % = 5.39. |

UV: $\lambda_{max1}$ = 292 nm ($\epsilon_1$: 30029)
$\lambda_{max2}$ = 231 nm ($\epsilon_2$: 17694).

**Example 49**

**(R,S)-1-(3,4-Dichlorophenyl)-6-methyl-3-(E)-[(3-cyclopropylamino-2-hydroxy)-propoxyimino]-1-(E)-heptene**

One proceeds as specified in Example 48 except that instead of pyrrolidine cyclopropyl amine (11.4 g, 0.2 mole) is used for the opening of the oxirane ring.
Yield: 29.1 g (72.8 %) of yellow oil.
2-(E)-Butenedioate (1/1) M.p.: 117 to 119 °C.

| Analysis for the formula $C_{24}H_{32}Cl_2N_2O_6$ (515.44): | | | | |
|---|---|---|---|---|
| Calculated: | C % = 55.94 | H % = 6.26 | Cl% = 13.76 | N % = 5.44 |
| Found: | C % = 55.80 | H % = 6.18 | Cl% = 13.49 | N % = 5.54. |

UV: $\lambda_{max1}$ = 292 nm ($\epsilon_1$: 31484)
$\lambda_{max2}$ = 231 nm ($\epsilon_2$: 18192).

**Example 50**

**(R,S)-1-(4-Chlorophenyl)-5-methyl-3-(E)-[(3-N-piperidinyl-2-hydroxy)-propoxyimino]-1-(E)-hexene**

One proceeds as specified in Example 47 except that 1-(4-chlorophenyl)-5-methyl-1-(E)-hexen-3-one-(E)-oxime (23.7 g, 0.1 mole) is used as oxime.
Yield: 29.9 g (84.8 %) of yellow oil.
Oxalate (1/1) M.p.: 118 to 120 °C.

| Analysis for the formula $C_{23}H_{33}ClN_2O_6$ (468.98): | | | | |
|---|---|---|---|---|
| Calculated: | C % = 58.90 | H % = 7.09 | Cl% = 7.57 | N % = 5.97 |
| Found: | C % = 58.62 | H % = 7.01 | Cl% = 7.42 | N % = 6.02. |

UV: $\lambda_{max1}$ = 224 nm ($\epsilon_1$: 13817)
$\lambda_{max2}$ = 292 nm ($\epsilon_2$: 32373).

**Example 51**

**(R,S)-1-(3,4-Dichlorophenyl)-6-methyl-3-(E)-[(3-N-piperidinyl-2-hydroxy)-propoxyimino]-1-(E)-heptene**

One proceeds as specified in Example 48 except that instead of pyrrolidine piperidine (17.0 g, 0.2 mole) is used for the opening of the oxirane ring.
Yield: 34.9 g (81.6 %) of yellow oil.
2-(E)-Butenedioate (1/1) M.p.: 134 to 135 °C.

| Analysis for the formula $C_{26}H_{36}Cl_2N_2O_6$ (543.49): | | | | |
|---|---|---|---|---|
| Calculated: | C % = 57.45 | H % = 6.68 | Cl% = 13.05 | N % = 5.16 |
| Found: | C % = 57.55 | H % = 6.73 | Cl% = 12.92 | N % = 5.25. |

UV: $\lambda_{max1}$ = 292 nm ($\epsilon_1$: 31689)
$\lambda_{max2}$ = 231 nm ($\epsilon_2$: 18796).

**Example 52**

**(R,S)-1-(4-Methoxyphenyl)-6-methyl-3-(E)-[(3-dimethylamino-2-hydroxy)-propoxyimino]-(E)-heptene**

One proceeds as specified in Example 47 except that instead of piperidine dimethylamine (13.5 g, 0.3 mole) is used for the opening of the oxirane ring.
Yield: 21.93 g (68.0 %) of yellow oil.
Oxalate (1/1) M.p.: 122 to 124 °C.

| Analysis for the formula $C_{22}H_{34}N_2O_7$ (438.53): | | | |
|---|---|---|---|
| Calculated: | C % = 60.25 | H % = 7.82 | N % = 6.38 |
| Found: | C % = 60.39 | H % = 7.88 | N % = 6.47. |

UV: $\lambda_{max1}$ = 224 nm ($\epsilon_1$: 13766)
$\lambda_{max2}$ = 292 nm ($\epsilon_2$: 32571).

**Example 53**

**(R,S)-1-(4-Chlorophenyl)-5-methyl-3-(E)-[(3-dimethylamino-2-hydroxy)-propoxyimino]-1-(E)-hexene**

One proceeds as specified in Example 50 except that instead of piperidine dimethylamine (13.5 g, 0.3 mole) is used for the opening of the oxirane ring.
Yield: 26.3 g (83.9 %) of yellow oil.
Oxalate (1/1) M.p.: 121 to 123 °C.

| Analysis for the formula $C_{20}H_{29}ClN_2O_6$ (428.92): | | | | |
|---|---|---|---|---|
| Calculated:<br>Found: | C % = 56.00<br>C % = 56.10 | H % = 6.82<br>H % = 6.79 | Cl% = 8.27<br>Cl% = 8.13 | N % = 6.53<br>N % = 6.61. |

UV: $\lambda_{max1}$ = 226 nm ($\epsilon_1$: 12910)
$\lambda_{max2}$ = 297 nm ($\epsilon_2$: 26141).

**Example 54**

**(R,S)-1-(4-Chlorophenyl)-6-methyl-3-(E)-[(3-N-methylamino-2-hydroxy)-propoxyimino]-1-(E)-heptene**

One proceeds as specified in Example 8 except that methylamine (6.2 g, 0.2 mole) is used for the opening of the oxirane ring.
Yield: 29.3 g (86.7 %) of yellow oil.

| Analysis for the formula $C_{22}H_{31}ClN_2O_6$ (454.96): | | | | |
|---|---|---|---|---|
| Calculated:<br>Found: | C % = 58.08<br>C % = 57.80 | H % = 6.87<br>H % = 6.84 | Cl% = 7.79<br>Cl% = 7.81 | N % = 6.16<br>N % = 6.13. |

UV: $\lambda_{max1}$ = 291 nm ($\epsilon_1$: 31299).

**Example 55**

**(R,S)-1-(4-Chlorophenyl)-6-methyl-3-(E)-[(3-N-hexyl-2-hydroxy)-propoxyimino]-1-(E)-heptene**

One proceeds as specified in Example 8 except that 1-aminohexane (20.2 g, 0.2 mole) is used for the opening of the oxirane ring.
Yield: 34.93 g (85.4 %) of viscous oil.
(E)-2-Butenedioate (2:1) M.p.: 119 to 121 °C.

| Analysis for the formula $C_{25}H_{39}ClN_2O_4$ (467.05): | | | | |
|---|---|---|---|---|
| Calculated:<br>Found: | C % = 64.29<br>C % = 64.70 | H % = 8.42<br>H % = 8.50 | Cl% = 7.59<br>Cl% = 7.32 | N % = 6.00<br>N % = 6.08. |

UV: $\lambda_{max1}$ = 291 nm ($\epsilon_1$: 32767).

**Example 56**

Tablet comprising 25 mg of active ingredient
The composition of one tablet is as follows

| | |
|---|---|
| active ingredient | 25.0 mg |
| corn starch | 97.0 mg |
| polyvinyl-pyrrolidone | 175.0 mg |
| magnesium stearate | 3.0 mg |
| | 300.0 mg |

The tablet is prepared as follows:

The active ingredient and the corn starch are admixed, then wetted with 10 to 15 % by weight of aqueous polyvinyl-pyrrolidone solution and the mixture is granulated, then dried at a temperature of 40 to 50 °C. The dry granules are rubbed through a sieve, mixed with magnesium stearate and tablets are prepared from the mixture.

The weight of one tablet is 300.0 mg.

**Example 57**

Tablet comprising 250 mg of active ingredient

The composition of one tablet is as follows:

| | |
|---|---|
| active ingredient | 250.0 mg |
| lactose | 270.0 mg |
| corn starch | 75.0 mg |
| magnesium stearate | 5.0 mg |
| | 600.0 mg |

The active ingredient, lactose and corn starch are wetted and mixed, granulated and dried at a temperature of 40 to 50 °C. The dry granules are rubbed through a sieve as described hereinabove, mixed with magnesium stearate, then tablets are formed.

The weight of one tablet is 600.0 mg.

**Example 58**

Dragée comprising 25 mg of active ingredient

The composition of one dragée core is as follows:

| | |
|---|---|
| active ingredient | 25.0 mg |
| corn starch | 245.0 mg |
| talc | 18.0 mg |
| gelatin | 8.0 mg |
| magnesium stearate | 4.0 mg |
| | 300.0 mg |

The active ingredient and corn starch are mixed, wetted with 10 % by weight aqueous gelatin solution, granules are formed from the wet mixture, then the granules are dried at a temperature of 40 to 50 °C. The dry granules are rubbed through a sieve, homogenized with talc and magnesium stearate and dragée cores of 300.0 mg are compressed from the mixture.

**Example 59**

Dragée comprising 50.0 mg of active ingredient

| | |
|---|---|
| active ingredient | 50.0 mg |
| lactose | 97.0 mg |
| polyvinyl-pyrrolidone | 2.0 mg |
| magnesium stearate | 1.0 mg |
| | 150.0 mg |

The granules are prepared as described hereinabove. The weight of the dragée cores is 150 mg.

The dragée cores are coated with a layer containing sugar and talc in a manner known per se. The dragée thus obtained is painted with non-toxic food paint to the desired colour and polished with bees-wax.

**Example 60**

Gelatin capsule comprising 5.0 mg of active ingredient
The composition of one gelatin capsule is as follows:

| | |
|---|---|
| active ingredient | 5.0 mg |
| corn starch | 40.0 mg |
| Aerosil® | 3.0 mg |
| magnesium stearate | 2.0 mg |
| | 50.0 mg |

The ingredients are homogenized and filled into gelatin capsules of suitable size.

**Example 61**

Gelatin capsule comprising 25.0 mg of active ingredient
The composition of one gelatin capsule is as follows:

| | |
|---|---|
| active ingredient | 25.0 mg |
| corn starch | 265.0 mg |
| Aerosil® | 6.0 mg |
| magnesium stearate | 4.0 mg |
| | 300.0 mg |

The ingredients are homogenized and filled into gelatin capsules of suitable size.

**Example 62**

Gelatin capsule comprising 50.0 mg of active ingredient
The composition of one gelatin capsule is as follows:

| | |
|---|---|
| active ingredient | 50.0 mg |
| lactose | 90.0 mg |
| Aerosil® | 6.0 mg |
| magnesium stearate | 4.0 mg |
| | 150.0 mg |

The ingredients are homogenized and filled into gelatin capsules of suitable size.

**Example 63**

Gelatin capsule comprising 250.0 mg of active ingredient
The composition of one gelatin capsule is as follows:

| | |
|---|---|
| active ingredient | 250.0 mg |
| lactose | 148.0 mg |
| magnesium stearate | 2.0 mg |
| | 400.0 mg |

The ingredients are homogenized and filled into gelatin capsules of suitable size.

**Example 64**

Injection solution comprising 25.0 mg of active ingredient. The composition of one ampoule is as follows:

| | |
|---|---|
| active ingredient | 25.0 mg |
| sodium chloride | 5.0 mg |

dissolved in 5 $cm^3$ of twice-distilled water.

The active ingredient and sodium chloride are dissolved in the necessary amount of twice-distilled water suitable for making injections. The solution is filtered, filled into ampoules and sterilized.

**Example 65**

Injection solution comprising 50.0 mg of active ingredient
The composition of one ampoule is as follows:

| | |
|---|---|
| active ingredient | 50.0 mg |
| sodium chloride | 10.0 mg |

The active ingredient and the sodium chloride are dissolved in the necessary amount of twice-distilled water, then filled into ampoules under sterile conditions.

**Example 66**

Suppository comprising 250 mg of active ingredient
The composition of one suppository is as follows:

| | |
|---|---|
| active ingredient | 250.0 mg |
| fatty acid glyceride | 750.0 mg |

The fatty acid glyceride is melted, the active ingredient is homogenized, then poured into a mould. One suppository weights 1000.0 mg and comprises 250.0 mg of active ingredient.

## Example 67

Drop comprising 5 % by weight of active ingredient

| | |
|---|---|
| active ingredient | 50.0 mg |
| sorbitol | 340.0 mg |
| polyethylene glycol | 100.0 mg |
| citric acid | 1.0 mg |
| sodium citrate | 3.0 mg |
| ion-free water | 505.0 mg |
| flavourant | 1.0 mg |
| | 1 000.0 mg |

The sorbitol, the active ingredient, citric acid and sodium citrate are dissolved in the aqueous solution of polyethylene glycol, then after dissolution of the solid materials the flavourant is added. The solution is filtered and filled into flasks supplied with a drop-dispenser.

## Claims

**1.** 1-(Phenyl)-3-[$\omega$-(amino)-alkoxyimino]-alk-1-ene derivatives of general formula

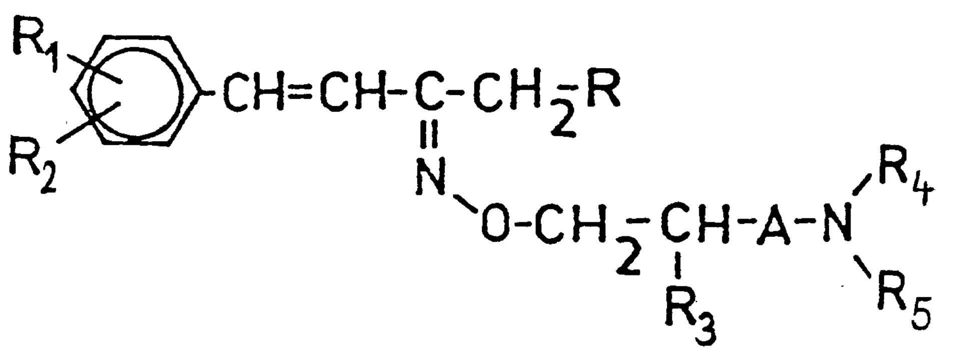

I;

wherein

R₁ and R₂ → see below

- $R_1$ and $R_2$ independently each stands for hydrogen, halogen or a $C_{1-4}$ alkoxy group or together represent a 3,4-methylenedioxy group,
- R stands for $C_{1-8}$ alkyl,
- $R_3$ represents hydrogen, $C_{1-4}$ alkyl or hydroxy,
- $R_4$ and $R_5$ independently each represents hydrogen, a $C_{1-12}$ alkyl group, optionally substituted by hydroxy, or a $C_{2-12}$ alkenyl or $C_{3-6}$ cycloalkyl group,

  or
- $R_4$ and $R_5$ together with the nitrogen atom to which they are attached form a 4- to 7-membered heterocyclic ring, optionally comprising an oxygen, sulfur or a further nitrogen atom, which latter optionally may carry a phenyl, benzyl or $C_{1-4}$ alkyl substituent

  and
- A represents a valency bond or a $-CH_2$-group,

as well as stereo and optically active isomers and their mixtures, acid-addition salts and quaternary ammonium derivatives thereof.

**2.** 1-(Phenyl)-3-[$\omega$-(amino)-alkoxyimino]-alk-1-ene derivatives according to claim 1, characterized in that the $C_{1-4}$ alkyl group which may be represented by $R_3$ and/or the $C_{1-4}$ alkyl substituent by which the optional further nitrogen atom of the 4- to 7-membered heterocyclic ring which may be formed by $R_4$ and $R_5$ together with the nitrogen atom to which they are attached may be substituted and/or the $C_{1-4}$ alkoxy group(s) which may be represented by $R_1$ and/or $R_2$ is/are such having from 1 to 3, particularly 1 or 2, carbon atom(s) and/or the $C_{1-12}$ alkyl group(s) which may be represented by $R_4$ and/or $R_5$ is/are such having from 1 to 8, particularly 1 to 4, carbon atom(s) and/or the $C_{1-8}$ alkyl group which

may be represented by R is such having from 1 to 7, particularly 1 to 6, carbon atom(s) and/or the $C_{2-12}$ alkenyl group(s) which may be represented by $R_4$ and/or $R_5$ is/are such having from 2 to 6, particularly 2 to 4, carbon atom(s) and/or the 4- to 7-membered heterocyclic ring which may be formed by $R_4$ and $R_5$ together with the nitrogen atom to which they are attached is such having from 5 to 7, particularly 5 or 6, carbon atoms, most particularly a morpholino, pyrrolidinyl, piperidinyl, piperazinyl, hexamethyleneimino or hexahydroazepinyl ring, and/or the halogen atom(s) which may be represented by $R_1$ and/or $R_2$ is/are chlorine, fluorine and/or bromine and/or the $C_{3-6}$ cycloalkyl group(s) which may be represented by $R_4$ and/or $R_5$ is/are such having 3, 5 or 6 carbon atoms.

**3.** 1-(Phenyl)-3-[ω-(amino)-alkoxyimino]-alk-1-ene derivatives according to claim 1 or 2, characterized in that

| | |
|---|---|
| $R_1$ and $R_2$ | independently each represents hydrogen or halogen, |
| $R_3$ | stands for hydrogen or hydroxy, |
| $R_4$ and $R_5$ | independently each represents $C_{1-4}$ alkyl |
| | or |
| $R_4$ and $R_5$ | together with the nitrogen atom to which they are attached form a piperidinyl or pyrrolidinyl group |
| | and |
| A and R | are as defined in claim 1 or 2. |

**4.** 1-[Phenyl]-5-[methyl]-3-(E)-[2'-(dimethylamino)-ethoxyimino]-1-(E)-hexene,
1-[4'-(chloro)-phenyl]-6-[methyl]-3-(E)-[3''-(N-{piperidinyl})-propoxyimino]-1-(E)-hexene,
1-(4'-(chloro)-phenyl]-3-(E)-[2''-(N-{piperidinyl})-ethoxyimino]-1-(E)-nonene,
(R,S)-1-[4'-(chloro)-phenyl]-6-[methyl]-3-(E)-[2''-(hydroxy)-3''-(piperidin-1'''-yl)-propoxyimino]-1-(E)-heptene,
1-[4'-(fluoro)-phenyl]-6-[methyl]-3-(E)-[3''-(N-{piperidinyl})-propoxyimino]-1-(E)-heptene,
1-[4'-(chloro)-phenyl]-6-[methyl]-3-(Z)-[2''-(N-{methyl}-pyrrolidin-2'''-yl)-ethoxyimino]-1-(E)-heptene
and
1-[4'-(chloro)-phenyl]-6-[methyl]-3-(E)-[3''-(N-{pyrrolidinyl})-propoxyimino]-1-(E)-heptene
as well as stereo and optically active isomers, acid-addition salts and quaternary ammonium derivatives thereof.

**5.** A process for preparing the compounds according to claims 1 to 4, characterized in that in a manner known per se

a) 1-(phenyl)-alk-1-ene-3-one or -3-thione derivatives of general formula

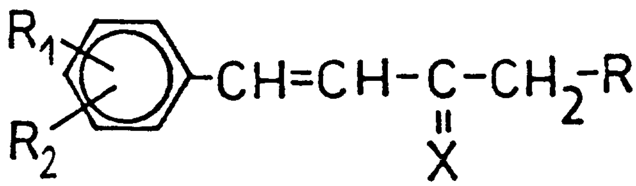

II,

wherein $R_1$, $R_2$ and R are as defined in claims 1 to 3 and X stands for oxygen or sulfur, are reacted with substituted alkyl-0-hydroxylamines of general formula

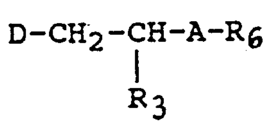

III,

wherein D represents a group of formula $H_2N$-0-,
$R_6$ stands for a group of formula

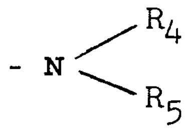 ,

in which latter $R_4$ and $R_5$ are as defined in claims 1 to 3 and $R_3$ and A are as defined in claims 1 to 3, or with the acid-addition salts thereof in the presence of a base

or

b) for preparing compounds of general formula I, wherein $R_3$ represents hydrogen or $C_{1-4}$ alkyl and R, $R_1$ , $R_2$ , $R_4$ and $R_5$ are as defined in claims 1 to 3, 1-(phenyl)-alk-1-ene-3-oxime derivatives of general formula

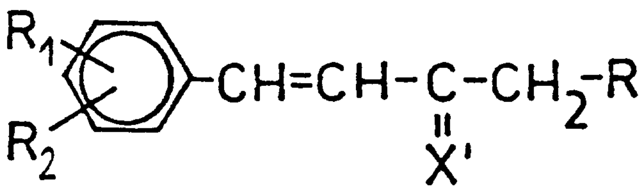

II',

wherein $R_1$ , $R_2$ and R are as defined in claims 1 to 3 and X' stands for a group of formula = N - OH, or acid-addition salts thereof are reacted with substituted alkylhalides of general formula

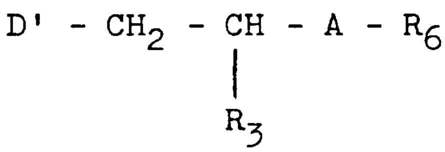

III'

wherein D' stands for halogen, $R_3$ represents hydrogen or $C_{1-4}$ alkyl, $R_6$ is a group of formula

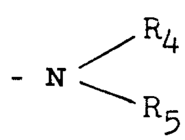 ,

in which latter $R_4$ and $R_5$ are as defined in claims 1 to 3 and A is as defined in claim 1, or with acid-addition salts thereof in the presence of a basic condensing agent

or

c) for preparing compounds of general formula I, wherein $R_3$ represents hydroxy, A stands for a group of formula - $CH_2$ - and R, $R_1$ , $R_2$ , $R_4$ and $R_5$ are as defined in claims 1 to 3, 1-(phenyl)-alk-1-ene-3-oxime derivatives of general formula II', wherein X' represents a group of formula = N - 0H and $R_1$ , $R_2$ and R are as defined in claims 1 to 3, or acid-addition salts thereof are reacted with 2,3-(epoxy)-propoxyhalides of general formula

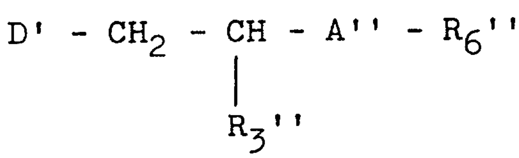

III'',

wherein D' represents halogen and A'', $R_6$'' and $R_3$'' together form a group of formula - $CH_2$ - 0 -, in the presence of a basic condensing agent, and the thus obtained 1-(phenyl)-3-[2',3'-(epoxy)-propoxyimino]-alk-1-enes of general formula

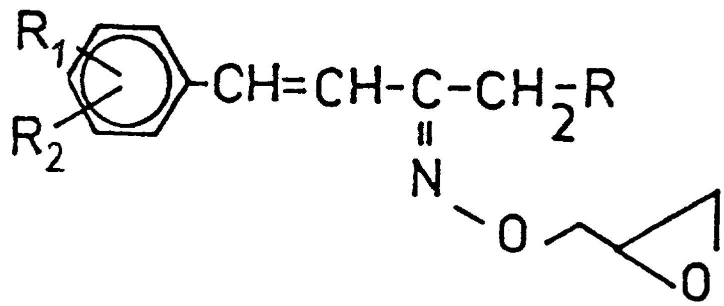

V,

wherein $R_1$, $R_2$ and R are as defined in claims 1 to 3, are reacted with amines of general formula

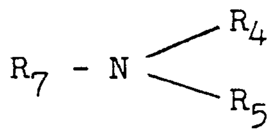

IV,

wherein $R_7$ stands for hydrogen, and $R_4$ and $R_5$ are as defined in claims 1 to 3 or in the presence of a basic agent with acid-addition salts of the former

or

d) for preparing compounds of general formula I, wherein $R_3$ represents hydrogen or $C_{1-4}$ alkyl and R, $R_1$, $R_2$, $R_4$, $R_5$ and A are as defined in claims 1 to 3, 1-(phenyl)-alk-1-ene-3-oxime derivatives of general formula II', wherein X' represents a group of formula = N - 0H and $R_1$, $R_2$ and R are as defined in claims 1 to 3, or acid-addition salts thereof are reacted with $\alpha,\omega$-dihalogenalkanes of general formula

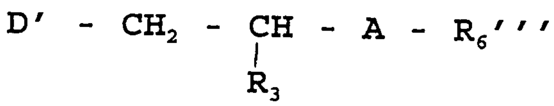

III''',

wherein D' stands for halogen, $R_3$ represents hydrogen or $C_{1-4}$ alkyl, $R_6'''$ means halogen and A is as defined in claim 1, in the presence of a basic condensing agent and the thus obtained 1-(phenyl)-3-[$\omega$-(halogen)-alkoxyimino]-alk-1-ene derivatives of general formula

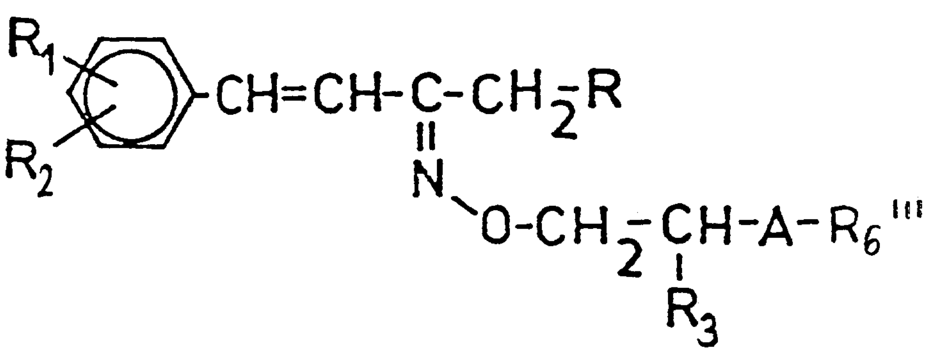

V' ,

wherein $R_1$, $R_2$, $R_3$, R and A are as defined in claims 1 to 3 and $R_6'''$ is as defined for formula III''', are reacted with amines of general formula

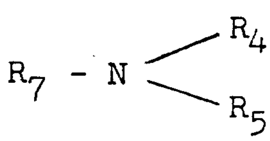

IV,

wherein $R_7$ stands for hydrogen, and $R_4$ and $R_5$ are as defined in claims 1 to 3, or with acid-addition salts thereof in the presence of a basic condensing agent

and optionally in a manner known per se 1-(phenyl)-3-[ω-(amino)-alkoxyimino]-alk-1-ene derivatives of general formula I thus obtained are converted into acid-addition salts or quaternary ammonium derivatives thereof or salts of 1-(phenyl)-3-[ω-(amino)-alkoxyimino]-alk-1-ene derivatives of general formula I thus obtained are converted into the free 1-(phenyl)-3-[ω-(amino)-alkoxyimino]-alk-1-ene derivative bases and/or into other acid-addition salts and/or mixtures or racemates of the above compounds of formula I are separated to the stereo and/or optically active isomers of the above compounds of formula I or the optically active isomers of the above compounds of formula I are racemised.

**6.** A process according to claim 5 a), characterized in that the reaction is carried out in inert solvents or in mixtures of such solvents, particularly in an aliphatic alcohol, pyridine or triethylamine or in a mixture thereof.

**7.** A process according to claim 5 b), characterized in that the reaction is carried out in inert solvents or mixtures of such solvents, particularly in an aliphatic alcohol, aromatic hydrocarbon, most particularly benzene or a homologue thereof, aliphatic or cyclic ether, most particularly dibutyl ether or tetrahydrofuran, or dimethyl acetamide or in a mixture thereof.

**8.** A process according to claim 5 c) or d), characterized in that the reaction of the 1-(phenyl)-alk-1-ene-3-oxime derivatives of general formula II' with the 1-(phenyl)-3-[2',3'-(epoxy)-propoxyimino]-alk-1-enes of general formula III'' or α,ω-dihalogenalkanes of general formula III''', respectively, is carried out in inert or relatively inert solvents, particularly water, an aromatic hydrocarbon, most particularly benzene or toluene, or dimethylformamide or in a mixture thereof.

**9.** Medicaments, characterized in that they contain as [an] active ingredient(s) at least one compound according to claims 1 to 4, suitably in admixture with at least one inert solid and/or liquid pharmaceutical carrier and/or diluent and/or excipient.

**10.** Use of the compounds of claims 1 to 4 for preparing medicaments, particularly having ulcus and gastric-acid-secretion inhibiting and anxiolytic effects.